# EUROPÄISCHE PATENTANMELDUNG

(11) **EP 3 893 527 A1**
(43) Veröffentlichungstag der Anmeldung: **13.10.2021**
(21) Anmeldenummer: 21163045.4
(22) Anmeldetag: 17.03.2021
(51) Int. Cl.: H04W 4/029, H04W 4/02, G16H 50/80, H04W 4/80, H04W 4/90, H04W 8/00, H04W 8/20, H04W 12/02, H04L 29/08, H04W 84/18, H04L 29/06, H04W 88/06

(54) **VERFAHREN ZUM REKONSTRUIEREN VON INFEKTIONSKETTEN**

(30) Priorität: 09.04.2020 DE 102020110065
(71) Anmelder: Scheidt & Bachmann GmbH, 41238 Mönchengladbach (DE)
(72) Erfinder: MILLER, Dr. Norbert, 41063 Mönchengladbach (DE); POMP, Georg, 41239 Mönchengladbach (DE); Rüßmann, Maximilian, 52062 Aachen (DE)
(74) Vertreter: Cohausz & Florack

(57) **Zusammenfassung**

Die Anmeldung betrifft ein Verfahren zum Rekonstruieren von Infektionsketten, mit den Schritten Empfangen, durch ein Ortungsgerät (102, 402, 502, 602), mindestens eines Nahfeld-Signals (126, 426) mindestens eines in Empfangsreichweite des Ortungsgeräts (102, 402, 502, 602) befindlichen mobilen Endgeräts (110, 116, 310, 410, 416) oder eines weiteren Ortungsgeräts (102, 402, 502, 602), Speichern mindestens einer Nutzerkennung in einer Datenbank (106, 306, 406) des Ortungsgeräts (102, 402, 502, 602), wobei die Nutzerkennung in dem mindestens einen Nahfeld-Signal (126, 426) enthalten ist, Aussenden, durch das Ortungsgerät (102, 402, 502, 602), eines Nahfeld-Advertising-Signals (126, 426), enthaltend zumindest ein Auswerteinformationsdatum, das zumindest eine potentielle Notwendigkeit einer Auswertung der mindestens einen gespeicherten Nutzerkennung angibt, wobei die Auswertung zumindest ein Vergleichen der mindestens einen in der Datenbank (106, 306, 406) des Ortungsgeräts (102, 402, 502, 602) gespeicherten Nutzerkennung mit mindestens einer in einer Infektionsdatenbank (132, 332, 432) gespeicherten Infektionskennung umfasst, die einem eine Infektionskrankheit aufweisenden Nutzer zugeordnet ist, Empfangen, durch das Ortungsgerät (102, 402, 502, 602), eines Antwortsignals auf das ausgesendete Nahfeld-Advertising-Signal von einem mobilen Auswerte-Endgerät (110, 310, 410), wobei das Antwortsignal ein Antwortdatum enthält, das zumindest eine Bereitschaft des mobilen Auswerte-Endgeräts (110, 310, 410) zum Veranlassen der Auswertung der mindestens einen gespeicherten Nutzerkennung angibt, und Übertragen der mindestens einen gespeicherten Nutzerkennung an das mobile Auswerte-Endgerät (110, 310, 410) zur Auswertung der mindestens einen gespeicherten Nutzerkennung, wenn das Ortungsgerät (102, 402, 502, 602) das Antwortsignal erhält und die Auswertung notwendig ist.

## Beschreibung

Die Anmeldung betrifft ein Verfahren zum Rekonstruieren von Infektionsketten. Die Anmeldung betrifft darüber hinaus ein Ortungsgerät, ein entsprechendes System, eine Anwendung und ein mobiles Auswerte-Endgerät.

Eine Infektionskrankheit (auch Infekt oder ansteckende Krankheit genannt) ist insbesondere eine durch Erreger (z.B. Bakterien, Pilze und/oder Viren) hervorgerufene Erkrankung bei Menschen. Der Erreger und damit die Infektionskrankheit kann von einer Person auf eine andere Person übertragen werden. Bei Infektionskrankheiten kann eine Übertragung der Krankheit grundsätzlich dann erfolgen, wenn sich zwei Personen temporär in einer räumlichen Nähe befinden, also ein räumlicher Kontakt stattgefunden hat.

Insbesondere die Covid-19-Epidemie bzw. -Pandemie, auch als Corona-Virus-Epidemie bezeichnet, aber auch andere Epidemien, haben gezeigt, dass es wichtig ist, zum Nachvollziehen von Infektionsketten, zum Eindämmen von Epidemien/Pandemien und zum Steuern von Shut-Down- und Ramp-Up-Maßnahmen, Personen zu identifizieren, die sich temporär mit einer infizierten Person in räumlicher Nähe befunden haben. Dies ist häufig dann nicht möglich, oder nur mit einem hohen Aufwand und hohen Messungenauigkeiten, wenn sich die infizierte Person in öffentlichen Räumen (z.B. öffentlich nutzbaren Fahrzeugen, Bahnhöfen, Restaurants etc.) bewegt hat.

Aus dem Stand der Technik ist es bekannt, dass zuständige Behörden und/oder Autoritäten zunehmend auf Daten aus Kommunikationsnetzen zugreifen und diese im Falle einer Epidemie nutzen. Dabei wird davon ausgegangen, dass in einer Bevölkerung eine große Zahl von Personen bzw. Nutzern ein mobiles Endgerät besitzen, wie ein Smartphone, die das Mobilfunk-Kommunikationsnetz nutzen.

Grundsätzlich kann der geographische Ort eines das Mobilfunk-Kommunikationsnetz nutzenden mobilen Endgeräts erfasst und damit ein Bewegungsprofil des entsprechenden Nutzers erstellt werden.

Die Lokalisierung von mobilen Endgeräten kann mittels einer Zuordnung eines jeweiligen Endgeräts zu einer Funkzelle des Mobilfunk-Kommunikationsnetzes bzw. Mobilfunknetz erfolgen.

Alternativ kann die Lokalisierung auf von einer vom mobilen Endgerät erfassten (eigenen) Geoposition auf Basis eines globales Navigationssatellitensystems, wie GPS, Galileo, Beidu (China) oder GLONASS (Russische Föderation), und einem Übertragen der zur Geoposition gehörigen Geopositionsdaten an das zentrale Erfassungssystem basieren.

Auf diese Weise entsteht im zentralen Erfassungssystem eine quasi-kontinuierliche Erfassung des geografischen Aufenthaltsorts (nahezu) aller mobilen Endgeräte.

Wenn im Falle einer ansteckenden Krankheit als infiziert bekannt gewordene Nutzer im zentralen Erfassungssystem in der zentralen Datenbank entsprechend gekennzeichnet werden, kann das Erfassungssystem die Sozialkontakte eines infizierten Nutzers anhand der Standortbestimmung und Standortverfolgung rekonstruieren und/oder in quasi-Echtzeit verfolgen sowie daraus welche auch immer gearteten Schlüsse ziehen oder Maßnahmen ableiten.

Neben Datenschutzproblemen bei den bekannten Verfahren ist insbesondere die geografische Auflösung häufig gering und damit problematisch.

So sind hinsichtlich der geografischen Auflösung die technischen Mittel zur Standortbestimmung mobiler Endgeräte nicht ausreichend genau genug. Insbesondere kann eine epidemiologisch relevante Distanz zwischen Personen typischerweise in einem Bereich kleiner als 10 m liegen, beispielsweise kleiner als 4 m, insbesondere kleiner als 2 m. Dies gilt insbesondere dann, wenn der hauptsächliche Übertragungsweg einer Infektionskrankheit eine Tröpfcheninfektion ist.

Die bekannten geografischen Standortbestimmungen von mobilen Endgeräten sind daher nicht genau genug, um eine epidemiologisch relevante Distanz zwischen zwei mobilen Endgeräten bestimmen zu können. Die räumliche Auflösung einer Funkzellenortung beträgt ein Vielfaches der epidemiologisch relevanten Distanz (zwischen 50 m und mehreren 100 m).

Ferner sind aus dem Stand der Technik Verfahren bekannt, die nicht auf geografische Standortbestimmung zurückgreifen, sondern Nahfeld-Signale, zum Beispiel Bluetooth-Signale, nutzen, um Nutzer, die sich temporär in einer räumlichen Distanz zu einem infizierten Nutzer bzw. einer infizierten Person befunden haben, zu identifizieren. So kann die räumliche Distanz mit einer höheren Genauigkeit bestimmt werden. Die Ortsbestimmung kann auch in Bereichen mit keiner ausreichenden GPS (oder dergleichen) Abdeckung erfolgen. Zudem werden bei der Bestimmung auch Höhenunterschiede berücksichtigt, beispielsweise in mehrstöckigen Gebäuden. Das Verfahren ermöglicht insbesondere einem Nutzer mittels seines mobilen Endgeräts zu bestimmen, ob der Nutzer potentiell in Kontakt mit einer infizierten Person gewesen ist. Auch ist es aufgrund der Verwendung von Nahfeld-Signalen nicht erforderlich, aus jeweiligen absoluten Standortdaten zweier Endgeräte die Distanz der Endgeräte zueinander zu berechnen.

Bekannte Tracking-Verfahren mittels Geopositionsdaten haben also die Nachteile eines geringen Datenschutzes, mangelnder Anonymität und unzureichend genauer Bestimmung von epidemologisch relevanten Kontakten.

Bekannte Verfahren unter Nutzung von Nahfeld-Signalen sind nachteilhaft, indem sie wegen fehlender Geopositionsdaten schlechte Nachvollziehbarkeit von Infektionsketten liefern, keine zentrale Datenbank zur Speicherung von Daten vorsehen, Daten nicht regelmäßig (sondern nur im Infektionsfall) an ein Backend laden und keine zentrales Auswerten im Backend unterstützen (das Backend ist reiner Informationsdienst). Darüber hinaus unterstützen bekannte Verfahren mit Nahfeld-Signalen keine Varianz des Datensignals, um beispielweise einen Sekundärkontakt in einer Infektionskette kenntlich zu machen. Ebenso lehren bekannte Verfahren nicht das Einrichten von Referenznutzern, die einen Zugriff auf Standortdaten ausdrücklich erlauben und damit das Nachvollziehen von Infektionsketten unterstützen.

Ein noch weiteres Problem besteht darin, dass trotz der weiten Verbreitung von mobilen Endgeräten in Form von Smartphones längst nicht jede Person über ein derartiges mobiles Endgerät verfügt. Insbesondere bei spezifischen Personengruppen, z.B. Senioren, Kinder, ist der Anteil der Personen, die über kein Smartphone verfügen, relativ hoch. Gerade bei der Gruppe der Senioren, die regelmäßig bei einer Epidemie zu den sogenannten Risikopersonen zählen, ist es wichtig, in einfacher Weise eine Möglichkeit zum Rekonstruieren von Infektionsketten zu schaffen.

Der Anmeldung liegt daher die Aufgabe zugrunde, ein Verfahren zur Verfügung zu stellen, das in einfacher Weise obige Defizite adressiert und insbesondere die Nachvollziehbarkeit von Infektionsketten bei gleichzeitig hohem Datenschutzniveau auch bei Personengruppen ermöglicht, bei denen der Anteil der Personen, die über kein Smartphone oder ein ähnliches mobiles Endgerät verfügen, relativ hoch ist.

Die Aufgabe wird gemäß einem ersten Aspekt der Anmeldung gelöst durch ein Verfahren zum Rekonstruieren von Infektionsketten nach Anspruch 1. Das Verfahren umfasst:
- Empfangen, durch ein Ortungsgerät, mindestens eines Nahfeld-Signals mindestens eines in Empfangsreichweite des Ortungsgeräts befindlichen mobilen Endgeräts oder eines weiteren Ortungsgeräts,
- Speichern mindestens einer Nutzerkennung in einer Datenbank des Ortungsgeräts, wobei die Nutzerkennung in dem mindestens einen empfangenen Nahfeld-Signal enthalten ist,
- Aussenden, durch das Ortungsgerät, eines Nahfeld-Advertising-Signals, enthaltend zumindest ein Auswerteinformationsdatum, das zumindest eine potentielle Notwendigkeit einer Auswertung der mindestens einen gespeicherten Nutzerkennung angibt, wobei die Auswertung zumindest ein Vergleichen der mindestens einen in der Datenbank des Ortungsgeräts gespeicherten Nutzerkennung mit mindestens einer in einer Infektionsdatenbank gespeicherten Infektionskennung umfasst, die einem eine Infektionskrankheit aufweisenden Nutzer zugeordnet ist,
- Empfangen, durch das Ortungsgerät, eines Antwortsignals auf das ausgesendete Nahfeld-Advertising-Signal von einem mobilen Auswerte-Endgerät, wobei das Antwortsignal ein Antwortdatum enthält, das zumindest eine Bereitschaft des mobilen Auswerte-Endgeräts zum Veranlassen der Auswertung der mindestens einen gespeicherten Nutzerkennung angibt, und
- Übertragen der mindestens einen gespeicherten Nutzerkennung an das mobile Auswerte-Endgerät zur Auswertung der mindestens einen gespeicherten Nutzerkennung, wenn das Ortungsgerät das Antwortsignal erhält und die Auswertung notwendig ist.

Indem im Gegensatz zum Stand der Technik anmeldungsgemäß insbesondere zusätzlich zu mobilen Endgeräten ein Ortungsgerät eingesetzt wird, das Nutzerkennungen von empfangenen Nahfeld-Signalen in einer Datenbank des Ortungsgeräts speichert und das eine Auswertung dieser gespeicherten Nutzerkennungen durch eine Interaktion mit einem mobilen Endgerät ermöglicht, wird in einfacher Weise ein Identifizieren von Personen (die kein mobiles Endgerät besitzen) ermöglicht, die sich temporär in einer räumlichen Distanz zu einer infizierten Personen befunden haben, insbesondere einer epidemiologisch relevanten Distanz.

Insbesondere können eine Vielzahl von Ortungsgeräten und mobilen Endgeräten und deren (automatisch stattfindenden) Kommunikationsvorgänge (wenn sie sich in Nahfeld-Reichweite zueinander befinden) verwendet werden.

Ein Ortungsgerät meint insbesondere ein mobiles oder stationäres Gerät mit mindestens einer Nahfeldschnittstelle und mindestens einem Datenspeicher, aber ohne eine eigene Fernkommunikationsschnittstelle. Anders ausgedrückt, weist ein Ortungsgerät insbesondere nur (mindestens) eine Nahfeldschnittstelle auf. Wesentlich ist insbesondere, dass ein anmeldungsgemäßes Ortungsgerät zumindest keine Telefoniefunktionalität aufweist. Zudem ist die Rechenleistung eines Ortungsgeräts in der Regel limitiert. Andererseits ist die Bedienung eines Ortungsgeräts einfach. Insbesondere ist (nahezu) keine Nutzerinteraktion mit dem Ortungsgerät erforderlich. Zudem kann ein kompaktes Gerät bereitgestellt werden, welches in einfacher Weise von einem Nutzer mit sich geführt werden kann. Hierdurch ist ein anmeldungsgemäßes Ortungsgerät insbesondere für Personengruppen, z.B. Senioren, Kinder, geeignet.

Ein mobiles Endgerät, das auch ein mobiles Auswerte-Endgerät bilden kann, umfasst vorliegend im Gegensatz zum Ortungsgerät eine eigene Fernkommunikationsschnittstelle, wie eine Mobilfunkschnittstelle. Insbesondere weist ein mobiles Endgerät eine Telefoniefunktionalität auf. Zudem weist ein mobiles Endgerät vorliegend mindestens eine Nahfeldschnittstelle auf.

Beispielhafte und nicht abschließende mobile Endgeräte sind Smartphones, Tablet-Computer, mobile Spielkonsolen, Laptops, Netbooks, Datenbrillen, Smart-Watches und ähnliche Wearables.

Wie bereits beschrieben wurde, weist ein Ortungsgerät mindestens eine Nahfeldschnittstelle auf, um Nahfeld-Signale zu empfangen und insbesondere auszusenden. Es versteht sich, dass ein Ortungsgerät zwei oder mehr unterschiedliche Nahfeldschnittstellen umfassen kann, für insbesondere eine entsprechende Anzahl an unterstützten unterschiedlichen Nahfeldtechnologien.

Gemäß dem anmeldungsgemäßen Verfahren ist insbesondere vorgesehen, dass aus einem empfangenen Nahfeld-Signal bzw. einer Nahfeld-Nachricht eine darin enthaltene Nutzerkennung extrahiert wird. Dies wird insbesondere bei jedem empfangenen Nahfeld-Signal durchgeführt.

Eine Nutzerkennung ist insbesondere eine (systemweit eineindeutig) Kennung, die einem Nutzer bzw. einem mobilen Endgerät des Nutzers oder einem Ortungsgerät des Nutzers zugeordnet ist. Bei einem Ortungsgerät wird die Nutzerkennung auch Ortungsgeräte-Kennung genannt. Beispielhafte und nicht abschließende Nutzerkennungen sind Endgerätekennungen, wie eine IMEI, Kommunikationsadressen des Endgeräts, Nutzernamen, eine MAC-Adresse und/oder eine eineindeutige Produktkennung einer auf dem mobilen Endgerät oder dem Ortungsgerät installierten Anwendung.

Vorzugsweis kann eine Nutzerkennung eine Kennzeichnung für die Anwendung enthalten, also einen Kennungsabschnitt, der (nur) von der Anwendung verarbeitbar ist. Ein entsprechender Kennungsabschnitt kann insbesondere durch die Anwendung einer Nutzerkennung hinzugefügt werden.

Alternativ oder zusätzlich kann eine Nutzerkennung einen Ländercode enthalten. Hierdurch ist insbesondere eine Bestimmung der Herkunft des entsprechenden Nutzers möglich. Ein entsprechender Ländercode kann insbesondere durch die Anwendung einer Nutzerkennung hinzugefügt werden.

Optional kann ferner vorgesehen sein, dass eine ausgesendete Nutzerkennung selbst verschlüsselt ist und/oder ein Zertifikat umfasst. Hierdurch kann insbesondere die Authentizität geprüft werden. Eine Verschlüsselung und/oder das Hinzufügen eines Zertifikats kann durch die Anwendung gesteuert werden. Auch kann für eine Prüfung der Authentizität der Nutzerkennung eine (verschlüsselte) Prüfsumme durch die Anwendung optional hinzugefügt werden. Empfangende Anwendungen können eine solche Nutzerkennung auf ihre Echtheit prüfen.

Anmeldungsgemäß wird zumindest die Nutzerkennung eines Nahfeld-Signals in einer (Nutzerkennungs-) Datenbank des Ortungsgeräts abgespeichert. Eine derartige Datenbank kann eine lokale auf dem Ortungsgerät vorhandene Datenbank sein. Das Speichern zumindest der empfangenen Nutzerkennungen kann insbesondere von einer Anwendung gesteuert werden, die auf dem Ortungsgerät installiert ist.

Da ein anmeldungsgemäßes Ortungsgerät insbesondere selbst keine Auswertung durchführen oder veranlassen kann, indem das Ortungsgerät die mindestens eine gespeicherte Nutzerkennung an das Backendsystem, insbesondere für eine Speicherung in der zentralen Infektionsdatenbank und/oder dezentralen Infektionsdatenbanken, etwa unmittelbar übertragen würde (oder von diesem die Infektionskennungen erhalten würde), wird anmeldungsgemäß vorgeschlagen, dass das Ortungsgerät in Reichweite der Nahfeldschnittstelle befindlichen mobilen Auswerte-Endgeräten signalisiert, dass zumindest potentiell eine Auswertung der in der Datenbank des Ortungsgeräts gespeicherten Nutzungskennungen erforderlich ist. Anmeldungsgemäß sendet das Ortungsgerät durch die Nahfeldschnittstelle (vorzugsweise regelmäßig) ein Nahfeld-Advertising-Signal aus, insbesondere gesteuert von der Anwendung, die auf dem Ortungsgerät installiert ist.

Mit anderen Worten: Das Ortungsgerät kann Kontaktdaten (also die Nutzerkennungen der mobilen Endgeräte und/oder anderen Ortungsgeräte, die temporär in seiner Nähe waren), zwar speichern, aber nicht verarbeiten. Das Ortungsgerät bedient sich der Fernkommunikations- und Rechenkapazität von in der Nähe befindlichen mobilen Endgeräten, um seine gespeicherten Kontaktdaten weiterverarbeiten zu lassen.

Das Nahfeld-Advertising-Signal des Ortungsgeräts kann zumindest ein Auswerteinformationsdatum enthalten, das zumindest eine potentielle Notwendigkeit einer Auswertung der mindestens einen gespeicherten Nutzerkennung angibt. Dies meint insbesondere, dass das Nahfeld-Advertising-Signal ein Auswerteinformationsdatum enthält, das angibt, dass das Ortungsgerät zumindest potentiell eine Auswertung der mindestens einen gespeicherten Nutzerkennung wünscht. Das Auswerteinformationsdatum kann beispielsweise in der Ortungsgeräte-Kennung integriert sein. Anders ausgedrückt, kann eine Ortungsgeräte-Kennung eine potentielle Notwendigkeit einer Auswertung der mindestens einen gespeicherten Nutzerkennung angeben. Es versteht sich, dass ein Auswerteinformationsdatum auch als zusätzliches Datum in dem Nahfeld-Advertising-Signal enthalten sein kann.

Eine Auswertung kann beispielsweise dann tatsächlich erforderlich sein, wenn mindestens eine Nutzerkennung in der Datenbank gespeichert ist. Alternativ oder zusätzlich kann eine Auswertung bei einem Überschreiten einer bestimmten Nutzerkennungsanzahl erforderlich sein, also wenn beispielsweise (in einem Zeitraum X) mehr als Y (entspricht der bestimmten Nutzerkennungsanzahl) Nutzerkennungen in der Datenbank gespeichert wurden. Es versteht sich, dass andere Kriterien für die Notwendigkeit einer Auswertung alternativ oder zusätzlich vorgegeben werden können, wie ein Zeitkriterium, das angibt, dass eine Auswertung zumindest nach einer bestimmten Zeitdauer (z.B. x Tage, x Stunden etc.) nach der letzten Auswertung notwendig ist.

Die Auswertung der mindestens einen in dem Ortungsgerät gespeicherten Nutzerkennung umfasst anmeldungsgemäß zumindest ein Vergleichen mit mindestens einer in einer (vorzugsweise in einer (zentralen) Infektionsdatenbank) gespeicherten Infektionskennung. Insbesondere für die vorbeschriebene Identifizierung von potentiell infizierten Personen erfolgt anmeldungsgemäß ein Vergleichen der mindestens einen in der Datenbank gespeicherten Nutzerkennung mit mindestens einer in einer (zentralen) Infektionsdatenbank gespeicherten Infektionskennung. Die zentrale Infektionsdatenbank kann insbesondere in einem Backendsystem implementiert sein. Auch kann die mindestens eine Datenbank eine dezentrale Datenbank (beispielsweise eines mobile Auswerte-Endgeräts) sein. In diesem Fall kann insbesondere das Backendsystem die Infektionskennungen an die mindestens eine dezentrale Datenbank verteilen. Es versteht sich, dass eine Mehrzahl unterschiedlicher Nutzerkennungen und/oder eine Mehrzahl unterschiedlicher Infektionskennungen gespeichert sein kann/können.

Eine Infektionskennung ist insbesondere auch eine Nutzerkennung (also beispielsweise eine Endgerätekennung, wie eine IMEI, Kommunikationsadresse des Endgeräts, Nutzername etc.), wobei diese Nutzerkennung einer Person zugeordnet ist, die (nachweislich) eine Infektionskrankheit aufweist. Infektionskennungen können in einer zentralen Datenbank gespeichert werden, beispielsweise gesteuert durch eine zentrale Instanz/Behörde. Vorzugsweise kann die zentrale Infektionsdatenbank und/oder mindestens eine dezentrale Infektionsdatenbank kontinuierlich mit Infektionskennungen infizierter Personen gefüllt werden, beispielsweise von z.B. Behörden/Ärzten.

Vorzugsweise kann die Nutzerkennung als eine anonymisierte Nutzerkennung gespeichert sein, und/oder die Infektionskennung kann als eine anonymisierte Infektionskennung gespeichert sein, wie ein jeweiliger Hash-Wert einer bestimmten Hash-Funktion. Der Datenschutz kann hierdurch verbessert werden.

Wenn sich ein mobiles Auswerte-Endgerät, also ein mobiles Endgerät, das eine vorbeschriebene Auswertung selbst durchführen kann und/oder eine Auswertung durch das Backendsystem (unmittelbar) veranlassen kann, in Reichweite des Ortungsgeräts befindet, sendet das Auswerte-Endgerät ein Antwortsignal auf das ausgesendete Nahfeld-Advertising-Signal aus. Auf einem Auswerte-Endgerät kann insbesondere eine Anwendung installiert sein, die ein Aussenden (automatisch) bei Empfang des genannten Nahfeld-Advertising-Signals bewirkt (und anschließend zumindest die Veranlassung der Auswertung steuert).

Das Antwortsignal, insbesondere ebenfalls ein Nahfeld-Advertising-Signal, enthält ein Antwortdatum, das zumindest eine Bereitschaft des mobilen Auswerte-Endgeräts (zumindest) zum Veranlassen der Auswertung der mindestens einen gespeicherten Nutzerkennung angibt. Es versteht sich, dass ein Auswerte-Endgerät kein Antwortsignal sendet, wenn eine Bereitschaft des mobilen Auswerte-Endgeräts (zumindest) zum Veranlassen der Auswertung nicht vorliegt, die entsprechende Anwendung beispielsweise temporär vom Nutzer deaktiviert ist.

Bei Erhalt des Antwortsignals und wenn (tatsächlich) eine Auswertung erforderlich ist (wie zuvor beschrieben wurde), überträgt das Ortungsgerät unter Nutzung einer Nahfeldschnittstelle die mindestens eine gespeicherte Nutzerkennung an das mobile Auswerte-Endgerät. Beispielsweise kann die Übertragung der (tatsächlich zu übertragenen) Nutzerkennungen (aus den gesamten gespeicherten Nutzerkennungen) abhängig sein von dem Empfangszeitpunt einer Nutzerkennung und der Inkubationszeit einer Infektionskrankheit.

Darüber hinaus kann vorzugsweise vorgesehen sein, dass die mindestens eine in der Datenbank gespeicherte Nutzerkennung nach einer (vorgegebenen) Speicherdauer gelöscht wird. Hierdurch kann der Speicherbedarf der Datenbank reduziert werden und insbesondere Datenschutzkriterien erfüllt werden.

Gemäß einer bevorzugten Ausführungsform des anmeldungsgemäßen Verfahrens kann das mindestens eine Nahfeld-Signal ein Nahfeld-Advertising-Signal sein.

Unter einem Nahfeld-Advertising-Signal ist vorliegend insbesondere ein Nahfeld-Signal zu verstehen, das von einem mobilen Endgerät oder einem Ortungsgerät ausgesendet werden kann und das von einem mobilen Endgerät oder einem Ortungsgerät empfangbar und insbesondere auswertbar ist, ohne dass eine (dauerhafte) Kopplung zwischen den genannten Geräten erforderlich ist. Insbesondere kann es auf mindestens einem von beispielsweise einer Mehrzahl von Advertising-Kanälen einer bestimmten Übertragungstechnologie ein Advertising-Signal, vorzugsweise in regelmäßigen Zeitabständen, (von jedem mobilen Endgerät und/oder Ortungsgerät) ausgesendet werden.

Im Stand der Technik wird ein Advertising-Signal eingesetzt, um eine dauerhafte Kommunikationsverbindung zwischen zwei Geräten herzustellen. Insbesondere wird ein Advertising-Signal für den ersten Kontakt zwischen den Geräten verwendet, insbesondere um die Anwesenheit und die grundsätzliche Bereitschaft eines Geräts für eine Kopplung einem weiteren (evtl. in Reichweite befindlichem) Gerät anzuzeigen. Nach Herstellung des ersten Kontakts wird anschließend auf einen anderen Kanal mit einer höheren Datenrate gewechselt, wenn eine dauerhafte Kommunikationsverbindung gewünscht ist. Die Daten werden dann nicht über Advertising-Signale ausgetauscht.

Vorzugsweise kann für ein Übertragen, durch das Ortungsgerät, der mindestens einen gespeicherten Nutzerkennung ein derartiger dauerhafter Nahfeld-Kommunikationskanal zwischen dem Ortungsgerät und dem mobilen Auswerte-Endgerät hergestellt werden.

Anmeldungsgemäß ist erkannt worden, dass ein Advertising-Signal vorteilhaft zur Anwesenheits-Signalisierung einer Nutzerkennung verwendet werden kann, da (sofern die entsprechende Nahfeldschnittstelle des mobilen Endgeräts aktiviert ist) ein solches Signal stets von einem Ortungsgerät empfangbar und auswertbar ist. Zudem enthält ein solches Signal in der Regel bereits eine Nutzerkennung des aussendenden mobilen Endgeräts bzw. des aussendenden Ortungsgeräts oder die Nutzerkennung des aussendenden mobilen Endgeräts bzw. des aussendenden Ortungsgeräts kann zumindest in einfacher Weise hinzugefügt werden.

Alternativ oder vorzugsweise zusätzlich kann das mindestens eine Nahfeld-Signal oder das mindestens eine Nahfeld-Advertising-Signal ein Bluetooth-Signal (insbesondere ein Bluetooth-Advertising-Signal) oder ein WLAN-Signal (insbesondere ein WLAN-Advertising-Signal) sein.

Besonders bevorzugt können das Nahfeld-Signal und das Nahfeld-Advertising-Signal jeweils ein Bluetooth Low Energy (BLE) Advertising-Signal sein. Ein BLE-Advertising-Signal kann insbesondere von nahezu jedem mobilen Endgerät, insbesondere mit einem marktüblichem Betriebssystem (z.B. Apple iOS, Google Android, Microsoft Windows Mobile, Microsoft Mobile Phone, Blackberry OS, Symbian OS, Firefox OS, Tizen, Aliyun OS) sowie mit dem anmeldungsgemäßen Ortungsgerät, empfangbar und auswertbar sein. Insbesondere kann ein Ortungsgerät in einfacher Weise für einen Empfang und eine Auswertung eines entsprechenden Signals konfiguriert werden, auch wenn beispielsweise die Rechenleistung des Ortungsgeräts gering ist.

Zudem weist die Bluetooth-Technologie den Vorteil auf, dass die Reichweite (z.B. auf ca. 10 m oder weniger) begrenzt ist, so dass mit einer hohen Sicherheit ein Advertising-Signal nur von mobilen Endgeräten und Ortungsgeräten empfangen wird, die sich in unmittelbarer Nähe zueinander befinden.

Bei anderen Varianten der Anmeldung kann das mindestens eine Advertising-Signal auch ein WLAN-Advertising-Signal, RFID-Advertising-Signal oder dergleichen sein. Es versteht sich, dass bei einer Variante auch zwei oder mehr unterschiedliche Advertising-Signale durch ein mobiles Endgerät ausgesendet werden können.

Gemäß einer weiteren Ausführungsform des anmeldungsgemäßen Verfahrens kann das Auswerteinformationsdatum ein Anfragedatum sein, wobei mit dem Anfragedatum durch das Ortungsgerät ein mobiles Auswerte-Endgerät für eine Auswertung angefragt werden kann. Insbesondere kann ein Anfragedatum, durch eine auf dem Ortungsgerät installierte Anwendung, hinzugefügt werden, wenn tatsächlich (und nicht nur potentiell) eine Auswertung notwendig ist. Die Notwendigkeit einer Auswertung kann beispielsweise bei Erfüllung eines vorgenannten Kriteriums detektiert werden. Beispielsweise durch Setzen eines Flags (oder dergleichen) in dem ausgesendeten Nahfeld-Advertising-Signal kann das Ortungsgerät aktiv eine Auswertung anfragen.

Bei diesem Ausführungsbeispiel kann das Antwortdatum eine Antwort-Bestätigung eines mobilen Auswerte-Endgeräts auf das Anfragedatum zum Veranlassen der Auswertung sein. Anders ausgedrückt, kann durch die Antwort-Bestätigung bestätigt werden, dass das Auswerte-Endgerät eine Auswertung nach einer Übertragung der mindestens einen Nutzerkennung zumindest veranlassen wird. Nach Erhalt der Antwort-Bestätigung kann in zuvor beschriebener Weise eine Übertragung durchgeführt werden.

Alternativ oder zusätzlich kann, gemäß einer weiteren Ausführungsform des anmeldungsgemäßen Verfahrens, das Auswerteinformationsdatum nur angeben, dass das Ortungsgerät (selbst) keine (unmittelbare) Auswertung veranlassen kann. Also anders ausgedrückt, kann das Auswerteinformationsdatum auch anzeigen, dass potentiell eine Auswertung der Nutzerkennungen erforderlich ist.

Wie bereits beschrieben wurde, kann dies bei einer Ausführungsform bereits aus der Ortungsgeräte-Kennung ableitbar sein. Alternativ oder zusätzlich kann beispielsweise ein Setzen eines Flags (oder dergleichen) in dem ausgesendeten Nahfeld-Advertising-Signal hierfür erfolgen. Ob tatsächlich eine Auswertung erforderlich bzw. gewünscht ist, ergibt sich aus dem Auswerteinformationsdatum bei dieser Ausführungsform insbesondere nicht.

Das Antwortdatum kann ein Auswerteanfragedatum eines mobilen Auswerte-Endgeräts sein. Mit dem Auswerteanfragedatum kann das mobile Auswerte-Endgerät das Ortungsgerät fragen, ob eine Auswertung (tatsächlich) notwendig bzw. gewünscht ist. Insbesondere kann das Antwortsignal ein vorbeschriebenes Nahefeld-Signal sein, bei dem (standardmäßig) ein Auswerteanfragedatum nur durch ein Ortungsgerät als Auswerteanfrage interpretierbar ist. In diesem Fall kann also zunächst das mobile Auswerte-Endgerät anfragen, ob tatsächlich eine Auswertung notwendig ist.

Wenn eine Auswertung (tatsächlich) erforderlich ist und insbesondere vor einem Übertragen der mindestens einen gespeicherten Nutzerkennung an das mobile Auswerte-Endgerät, kann das Ortungsgerät eine Bestätigungsantwort senden, beispielsweise durch Aussenden eines Nahfeld-Advertising-Signals. Die Bestätigungsantwort kann ein Bestätigungsantwortdatum enthalten. Hierdurch kann insbesondere angezeigt werden, dass die Auswertung notwendig bzw. gewünscht ist. Anschließend kann in zuvor beschriebener Weise eine Übertragung durchgeführt werden.

Wie bereits beschrieben, kann das Auswerten der mindestens einen übertragenen Nutzerkennung ferner die Schritte umfassen:
- Vergleichen, in einem Vergleichsschritt, der mindestens einen übertragenen Nutzerkennung mit mindestens einer in der (zentralen) Infektionsdatenbank gespeicherten Infektionskennung, die einem eine Infektionskrankheit aufweisenden Nutzer zugeordnet ist,
- Detektieren einer Korrespondenz zwischen der mindestens einen übertragenen Nutzerkennung und der mindestens einen gespeicherten Infektionskennung und
- Übertragen, basierend auf der detektierten Korrespondenz, einer Infektionsinformation an das Ortungsgerät.

In dem Vergleichsschritt kann insbesondere detektiert werden, ob eine (eindeutige) Korrespondenz zwischen der mindestens einen übertragenen Nutzerkennung des Ortungsgeräts und der mindestens einen gespeicherten Infektionskennung vorliegt (oder nicht). Eine Korrespondenz liegt insbesondere bei einer Identität der verglichenen Kennungen vor. Auch kann eine Korrespondenz vorliegen, wenn zwei Kennungen einander eindeutig zugeordnet werden können, und damit als von der gleichen Person stammend identifiziert werden können.

Gemäß dieser Ausführungsform ist insbesondere ein Übertragen einer Infektionsinformation an das Ortungsgerät vorgesehen, vorzugsweise in Form eines Vergleichsergebnisdatensatzes, enthaltend zumindest das Vergleichsergebnis, wie mindestens eine festgestellte Korrespondenz. Bei Erhalt einer Infektionsinformation kann insbesondere ein Ausgeben der Infektionsinformation durch das Ortungsgerät erfolgen. Zumindest wenn eine Korrespondenz detektiert wurde, erfolgt ein Übertragen und insbesondere ein Ausgeben. Eine detektierte Korrespondenz gibt insbesondere an, dass der Nutzer des Ortungsgeräts sich in räumlicher Nähe zu einer infizierten Person befunden hat. Der Nutzer kann sich demnach bei der infizierten Person angesteckt haben.

Ein Vergleichsergebnisdatensatz kann vorzugsweise neben dem Vergleichsergebnis weitere Daten umfassen, wie eine Handlungsempfehlung und/oder mindestens einen (vordefinierten) Notfallkontakt.

Vorzugsweise kann das Ausgeben einer Infektionsinformation durch das Ortungsgerät ein Ausgeben (z.B. ein Anzeigen einer Nachricht auf einem Display des Ortungsgeräts, Aussenden einer Push-Nachricht etc.) des Vergleichsergebnisses umfassen. Ferner kann ein Ausgeben von mindestens einer Handlungsempfehlung und/oder mindestens einem (vordefinierten) Notfallkontakt erfolgen.

Alternativ oder zusätzlich kann auch ein Übertragen einer entsprechenden Nachricht an die zentrale Instanz erfolgen. Die zentrale Instanz kann dann wiederum entsprechende Maßnahmen ergreifen.

Gemäß einer weiteren bevorzugten Ausführungsform des anmeldungsgemäßen Verfahrens kann zu jeder Nutzerkennung mindestens eine weitere Angabe in der Datenbank des Ortungsgeräts gespeichert werden. Die weitere Angabe kann ausgewählt sein aus der Gruppe, umfassend:
- eine Empfangszeitangabe über den Empfangszeitpunkt des Nahfeld-Signals,
- eine Zeitdauerangabe über die Empfangsdauer von Nahfeld-Signalen mit gleicher Nutzerkennung,
- eine Distanzangabe über die Distanz zwischen dem Ortungsgerät und dem mobilen Endgerät oder dem weiteren Ortungsgerät,
- eine Infektionsangabe abhängig von dem in dem Vergleichsschritt durchgeführten Vergleich.

Vorzugsweise können zwei, besonders bevorzugt sämtliche der genannten Angaben/Daten für jede empfangene Nutzerkennung gespeichert werden. Dies meint insbesondere, dass jeder Nutzerkennung eine entsprechende Angabe zugeordnet sein kann und dass diese Daten abgespeichert werden, beispielswiese in Tabellenform. Es versteht sich, dass weitere Daten für jede Nutzerkennung gespeichert werden.

Insbesondere kann zu jeder empfangenen Nutzerkennung der Empfangszeitpunkt in Form eines Zeitstempels (z.B. 26.03.2020, 15:41 Uhr) des Nahfeld-Signals erfasst und gespeichert werden. Dies kann bevorzugt ein Zuordnen des erfassten Empfangszeitpunkts zu einem bestimmten Empfangszeitbereich umfassen. Beispielsweise kann die vergangene Zeit in mehrere Empfangszeitbereiche unterteilt werden (z.B. eine wochenweise oder tageweise Unterteilung). Abhängig von dem erfassten Empfangszeitpunkt kann eine Zuordnung zu einem bestimmten Empfangszeitbereich erfolgen. Der bestimmte Empfangszeitbereich kann dann als Empfangszeitangabe gespeichert werden. Eine Abspeicherung des tatsächlichen Empfangszeitdatums kann unterbleiben. Neben einer einfacheren Datenbankstruktur und einer einfacheren Auswertung der gespeicherten Daten, kann der Datenschutz verbessert werden. Beispielsweise werden Rückschlüsse auf Kontakte und Personen verhindert.

Vorzugsweise zusätzlich kann eine Zeitdauerangabe über die Empfangsdauer der wiederholend empfangenen Nahfeld-Signale mit gleicher Nutzerkennung gespeichert werden. Insbesondere kann die Zeitdauer gemessen werden, während die Nahfeld-Signale bzw. Nahfeld-Nachrichten wiederholt bzw. nahezu kontinuierlich mit der gleichen Nutzerkennung empfangen wurden.

Dies kann bevorzugt das Zuordnen zu einem bestimmten Zeitdauerbereich umfassen. Beispielsweise können unterschiedliche Zeitdauerbereiche (z.B. x < 1 min, 1 min < x < 5 min, 5 min < x < 15 min, 15 min < x < 60 min etc.) vordefiniert werden. Abhängig von der tatsächlichen Empfangsdauer kann eine Zuordnung zu einem vordefinierten Zeitdauerbereich erfolgen. Der bestimmte Zeitdauerbereich kann dann als Zeitdauerangabe gespeichert werden. Eine Abspeicherung der tatsächlichen Empfangsdauer kann unterbleiben. Neben einer einfacheren Datenbankstruktur und einer einfacheren Auswertung der Daten, kann der Datenschutz verbessert werden. Beispielsweise werden Rückschlüsse auf Kontakte und Personen verhindert.

Besonders bevorzugt kann eine Distanzangabe über die Distanz zwischen dem Ortungsgerät und dem mobilen Endgerät oder dem weiteren Ortungsgerät gespeichert werden. Insbesondere kann die Distanz durch ein Distanzbestimmungsmodul des Ortungsgeräts bestimmt werden, basierend auf dem empfangenen Nahfeld-Signal, enthaltend die Nutzerkennung. Vorzugsweise kann die Distanz basierend auf dem RSSI (Received Signal Strength Indicator) bestimmt werden. Der RSSI stellt insbesondere einen Indikator für die Empfangsfeldstärke des empfangenen Informationssignals dar, aus der die Distanz zum sendenden Gerät zumindest abgeschätzt werden kann.

Wenn mehrere Nahfeld-Signale mit der gleichen Nutzerkennung, insbesondere mit unterschiedlichen Distanzwerten, empfangen werden, kann eine Minimumbestimmung erfolgen. Anders ausgedrückt kann als Distanzangabe in Bezug auf eine bestimmte Kontaktperson die kürzeste Distanz aus der Mehrzahl von Distanzwerten bestimmt werden.

Das Speichern kann bevorzugt das Zuordnen zu einem Distanzbereich von einer Mehrzahl von vordefinierten Distanzbereichen (z.B. x < 0,5 m, 0,5 m < x < 2 m, 2 m < x < 4 m, x> 4 m) umfassen. Die Distanzbereiche können insbesondere von der epidemiologisch relevanten Distanz einer Infektionskrankheit abhängen. Abhängig von der tatsächlichen Distanz kann eine Zuordnung zu einem vordefinierten Distanzbereich erfolgen. Der bestimmte Distanzbereich kann dann als Distanzangabe gespeichert werden. Eine Abspeicherung der tatsächlichen Distanz kann unterbleiben. Neben einer einfacheren Datenbankstruktur und einer einfacheren Auswertung der gespeicherten Daten, kann der Datenschutz verbessert werden. Beispielsweise werden Rückschlüsse auf Kontakte und Personen verhindert.

Darüber hinaus kann eine Infektionsangabe abhängig von dem in dem Vergleichsschritt durchgeführten Vergleich gespeichert werden. Wenn das Vergleichsergebnis beispielsweise anzeigt, dass die Nutzerkennung zu einer infizierten Person gehört, kann ein entsprechendes Datum (z.B. "infizierte Person") gespeichert werden. Bei einem negativen Vergleichsergebnis kann ein entsprechendes Datum (z.B. "nicht infizierte Person") gespeichert werden. Wenn noch kein Abgleich stattgefunden hat, kann das Datum fehlen.

Besonders bevorzugt kann, gemäß einer Ausführungsform, eine Geopositionsangabe über die geographische Position des aussendenden mobilen Endgeräts bei einem Empfang des Nahfeld-Signals in der Datenbank des Ortungsgeräts gespeichert werden. Insbesondere kann eine Anwendung dem Nutzer optional die Einstellung ermöglichen, dass bei einem Aussenden eines Nahfeld-Signals die (augenblickliche) geographische Position des aussendenden mobilen Endgeräts bestimmt wird, beispielsweise auf Basis eines globalen Navigationssatellitensystems, wie GPS, Galileo, Beidu (China) oder GLONASS (Russische Föderation), und dem Nahfeld-Signal hinzugefügt wird. Die Geopositionsdaten, also insbesondere die geographischen Koordinaten, können als zusätzliche Angabe in der Datenbank zusammen mit der Nutzerkennung des Nahfeld-Signals gespeichert werden.

Um Infektionsketten besser nachzuvollziehen, ist anmeldungsgemäß erkannt worden, dass geographische Daten bzw. Geopositionsdaten wichtig sind. Insbesondere liefert ein reiner Rückgriff auf Nahfeld-Daten keinen Rückschluss auf die jeweilige absolute geografische Position des sendenden mobilen Endgeräts bzw. des entsprechenden Nutzers.

Gemäß dieser Ausführungsform wird vorgeschlagen, dass ein mobiles Endgerät zu einem mobilen Referenzendgerät (bzw. sein Nutzer zu einem Referenznutzer) werden kann. Zum mobilen Referenzendgerät kann das mobile Endgerät beispielsweise dadurch werden, dass in der Anwendung unter "Optionen" (oder dergleichen) eine Ortung des mobilen Endgeräts freigegeben wird, beispielswiese anhand von Daten eines globales Navigationssatellitensystems und/oder Mobilfunkdaten. In der Folge können, vorzugsweise bei jedem Aussenden eines Nahfeld-Signals, die Geopositionsdaten des sendenden mobilen Endgeräts als Geopositionsangaben hinzugefügt und anschließend von dem Ortungsgerät in der Datenbank gespeichert werden.

Vorzugsweise kann nur im Falle einer Übereinstimmung der verglichenen Kennungen bei dem beschriebenen Abgleich zwischen der Datenbank des Referenzendgeräts und der (zentralen) Infektionsdatenbank ein Zugriff auf die Geopositionsangabe erfolgen, zum Beispiel durch das Backendsystem bzw. eine zentrale Instanz. Andernfalls ist der Zugriff auf die gespeicherten Geopositionsangaben gesperrt. Ein Zugriff auf die genannte Geopositionsangabe erlaubt vorteilhafterweise, dass nachvollzogen werden kann, wo sich der infizierte Nutzer mit dem Referenznutzer getroffen hat.

Das (zentrale) Backendsystem bzw. die zentrale Instanz kann, basierend auf der Geopositionsangabe, ggf. weitere Kontaktpersonen, mit denen der Referenznutzer nach dem Kontakt zur infizierten Person Kontakt gehabt hat, über einen sogenannten Sekundärkontakt informieren (ggf. nur wenn der Kontakt des Referenznutzers zur infizierten Person gewisse Kriterien, wie eine ausreichende Distanz (z.B. < 4 m) erfüllt).

Über die Geopositionsangabe des Referenzendgeräts und die dokumentierten Sekundärkontakte (darunter können auch wiederum Referenznutzer mit entsprechenden Geopositionsangaben sein) können sich Infektionsketten in einfacher Weise nachweisen lassen.

Insbesondere kann das Ausgeben einer Infektionsinformation ein Speichern einer entsprechenden Infektionsangabe in der Datenbank des Ortungsgeräts umfassen.

In der Datenbank des Ortungsgeräts können die vorbeschriebenen Daten in einer Tabellenform gespeichert werden, wie beispielhaft in Tabelle 1 dargestellt.

**Tabelle 1**

| I**Ds** | **Empfangszeita.** | **Zeita.** | **Distanza.** | **Infektionsa.** | **Geoposition** |
|---|---|---|---|---|---|
| IDA | 25.03.20, 16:38 | 30 s | 3 m | Nein | 51° 10'41.47" N 6° 27' 18.317" E |
| IDB | 23.03.20, 17:45 | 5 min, 30 s | 1,5 m | Nein | 51° 11'45.866" N 6° 26' 45.316" E |
| ··· | ··· | ··· | ··· | ··· | |

In der Tabelle 1 sind beispielhafte Nutzerkennungen (IDs), Empfangszeitangaben (Empfangszeita.), Zeitdauerangaben (Zeitdauera.), Distanzangaben (Distanza.) und Infektionsangaben (Infektionsa.) sowie Geopositionsdaten (Geoposition) dargestellt.

Gemäß einer weiteren Ausführungsform des anmeldungsgemäßen Verfahrens können die gespeicherten Daten/Angaben verschlüsselt gespeichert werden. Dies erhöht noch weiter die Datensicherheit.

Um die Datenbankgröße bei dem Ortungsgerät klein zu halten, können vorzugsweise nur potentiell relevante Daten in der Datenbank gespeichert werden. Nutzerkennungen von Endgeräten oder Ortungsgeräten, von denen klar ist, dass ein Kontakt nicht epidemologisch relevant war (beispielsweise durch zu große Entfernung und/oder zu kurze Kontaktdauer), können beispielsweise erst gar nicht gespeichert werden.

Gemäß einer bevorzugten Ausführungsform des anmeldungsgemäßen Verfahrens kann mindestens ein Speicherkriterium vorgegeben sein. Das Speichern zumindest der Nutzerkennung (vorzugsweise auch der vorbeschriebenen weiteren Angaben) in der Datenbank kann erfolgen basierend auf dem mindestens einen vorgegebenen Speicherkriterium. Das mindestens eine Speicherkriterium ist insbesondere eine Angabe, wann eine Nutzerkennung (und z.B. mindestens eine weitere Angabe) gespeichert wird (oder wann eine Nutzerkennung nicht gespeichert wird).

Vorzugsweise kann, gemäß einer weiteren Ausführungsform des anmeldungsgemäßen Verfahrens, das mindestens eine Speicherkriterium eine Mindestzeitdauer umfassen, die eine Mindestempfangszeitdauer für den wiederholenden Empfang von Nahfeld-Signalen mit gleicher Nutzerkennung angibt. Insbesondere kann das Speicherkriterium festlegen, dass ein Speichern nur dann erfolgt, wenn die bestimmte Zeitdauerangabe die (vorbestimmte) Mindestempfangszeitdauer (z.B. 10 s, 30 s etc.) übersteigt. Ansonsten kann eine Speicherung (komplett) unterbleiben.

Alternativ oder bevorzugt zusätzlich kann das mindestens eine Speicherkriterium eine Höchstdistanz umfassen, die eine maximale Distanz zwischen dem Ortungsgerät und dem mindestens einen weiteren Gerät angibt. Insbesondere kann das Speicherkriterium festlegen, dass ein Speichern nur dann erfolgt, wenn die bestimmte Distanzangabe die (vorbestimmte) Höchstdistanz (z.B. 5 m, 10 m etc.) nicht übersteigt. Bei der Vorgabe der Höchstdistanz können insbesondere Messtoleranzen und die epidemiologisch relevante Distanz der mindestens einen Infektionskrankheit berücksichtigt werden.

Gemäß einer bevorzugten Ausführungsform des anmeldungsgemäßen Verfahrens kann eine von der Zeitdauerangabe und der Distanzangabe abhängige Infektionswahrscheinlichkeitsfunktion vorgesehen sein. Die Infektionswahrscheinlichkeitsfunktion kann insbesondere angeben, wie hoch die Wahrscheinlichkeit einer Ansteckung geschätzt wird, beispielsweise zwischen einem maximalen Infektionswahrscheinlichkeitsfaktor (z.B. 1 oder 100 %) und einem minimalen Infektionswahrscheinlichkeitsfaktor (0 bzw. 0 %). Die Infektionswahrscheinlichkeitsfunktion hängt insbesondere von den zu einer Nutzerkennung zugehörigen Zeitdauerangabe und Distanzangabe ab.

Vorzugsweise kann ein Infektionswahrscheinlichkeitsfaktor für eine Nutzerkennung durch die Infektionswahrscheinlichkeitsfunktion und den für die Nutzerkennung bestimmten Zeitdauerangabe und Distanzangabe ermittelt (z.B. berechnet) werden. Um beispielsweise die Datenbankgröße gering zu halten, kann vorzugsweise nur die Nutzerkennung und der zughörige Infektionswahrscheinlichkeitsfaktor in der Datenbank gespeichert werden. Insbesondere kann auf eine Speicherung der Zeitdauerangabe und der Distanzangabe verzichtet werden.

Die Infektionswahrscheinlichkeitsfunktion kann beispielsweise wie folgt gebildet sein: f = Nähe (t) einer anderen Nutzerkennung, z.B. das Zeitintegral über die Nähe/Distanz zu dieser Nutzerkennung bzw. dem entsprechenden mobilen Endgerät.

Gemäß einer weiteren Ausführungsform des anmeldungsgemäßen Verfahrens kann das mindestens eine weitere Speicherkriterium ein Infektionswahrscheinlichkeitsfaktorbereich sein. Ein Speichern der Nutzerkennung und des zughörigen Infektionswahrscheinlichkeitsfaktors kann nur dann erfolgen, wenn der Infektionswahrscheinlichkeitsfaktor in dem Infektionswahrscheinlichkeitsfaktorbereich liegt, also beispielsweise zwischen 0,1 und 1 (bei einem maximalen Infektionswahrscheinlichkeitsfaktor von 1 und einem minimalen Infektionswahrscheinlichkeitsfaktor von 0) oder zwischen 10 % und 100 % (bei einem maximalen Infektionswahrscheinlichkeitsfaktor von 100 % und einem minimalen Infektionswahrscheinlichkeitsfaktor von 0 %).

Darüber hinaus kann, gemäß einer weiteren Ausführungsform des anmeldungsgemäßen Verfahrens, zu jeder Infektionskennung mindestens eine weitere Infektionsangabe in der (zentralen) Infektionsdatenbank gespeichert werden. Die weitere Infektionsangabe kann ausgewählt sein aus der Gruppe, umfassend:
- Infektionsart (über die Art der Infektionskrankheit),
- Infektionszeitpunkt.

Vorzugsweise können beide Infektionsangaben zusammen mit der jeweiligen Infektionskennung gespeichert werden.

Die Infektionsart ermöglicht insbesondere eine Identifizierung der Infektionskrankheit des entsprechenden Nutzers. Dies ermöglicht insbesondere eine Bestimmung der Inkubationszeit. Bei anderen Varianten können auch beide Angaben (Infektionsart und Inkubationszeit) gespeichert werden.

Der Infektionszeitpunkt gibt insbesondere den Zeitpunkt an, an dem sich die Person (voraussichtlich) mit der Krankheit infiziert hat. Es versteht sich, dass hierbei Toleranzwerte berücksichtigt werden können und beispielsweise zwei (oder mehr) Werte angegeben sein können (z.B. frühester und spätester Infektionszeitpunkt). Alternativ oder zusätzlich kann der Infektionszeitpunkt der Zeitpunkt sein, an dem die Infektionskrankheit nachgewiesen wurde.

In der (zentralen) Infektionsdatenbank können die vorbeschriebenen Daten/Angaben in Tabellenform gespeichert werden, wie beispielhaft in Tabelle 2 dargestellt.

**Tabelle 2**

| **Infektionskennung (ID_{K})** | **Infektionszeitpunkt** | **Infektionsart** |
|---|---|---|
| ID_{K} C | 22.03.20 | COV19 |
| ID_{K} B | 24.03.20 | COV19 |
| ··· | ··· | ··· |

Bei den Beispielen nach Tabelle 1 und 2 wird in einem Vergleichsschritt, beispielsweise eine Identität zwischen der Nutzerkennung ID B und der Infektionskennung ID_{K} B, detektiert.

Anschließend kann eine Infektionsinformation, insbesondere umfassend die Angabe, dass der Nutzer des Ortungsgeräts Kontakt mit einer infizierten Person hatte, übertragen und vorzugsweise ausgegeben werden. Der Nutzer des Ortungsgeräts und/oder eine zentrale Instanz können weitere Maßnahmen veranlassen, wie das Durchführen eines Infektionstests zur Feststellung, ob sich auch der Nutzer des Ortungsgeräts infiziert hat.

Ferner kann gemäß einer Ausführungsform vorgesehen sein, dass z.B. in der Datenbank des Ortungsgeräts zu einer Infektionsart ein Infektionskenner hinterlegt ist, der dem Nutzer des Ortungsgeräts zugeordnet ist. Der Kenner kann mindestens eine Angabe über vergangene Infektionskrankheiten des Nutzers angeben, beispielsweise eine Immunität gegenüber einer bestimmten Infektionskrankheit. Dies kann in dem Vergleichsschritt berücksichtigt werden. Insbesondere kann ein Abgleich nur mit Infektionskennungen mit einer Infektionsart durchgeführt werden, gegen die der Nutzer nicht immun ist.

Auch können in der Datenbank des Ortungsgeräts optional Impfangaben (z.B. entsprechend einem Impfpass des Nutzers) hinterlegt sein. Diese Angaben können jeweils ein Ablaufdatum des Impfschutzes enthalten, beispielsweise mit Erinnerung an eine fällige nächste Impfung durch die Anwendung.

Gemäß einer bevorzugten Ausführungsform des anmeldungsgemäßen Verfahrens kann eine zentrale Infektionsdatenbank in einem Backendsystem angeordnet sein. Ein Backendsystem kann von einem oder mehreren (verteilt angeordneten) Server/n gebildet sein. Ein Backendsystem ist insbesondere entfernt von dem mindestens einen Ortungsgerät angeordnet.

Das Verfahren kann ferner umfassen:
- Übertragen, durch das mobile Auswerte-Endgerät, eines Nutzerkennungsdatensatzes an das Backendsystem, wobei der Nutzerkennungsdatensatz zumindest die mindestens eine übertragene Nutzerkennung enthält,
- Durchführen, durch das Backendsystem, des Vergleichsschritts, und
- Übertragen, durch das Backendsystem, zumindest eines Vergleichsergebnisdatensatzes als Infektionsinformation (mittelbar) an das Ortungsgerät.

Anders ausgedrückt wird in dem vorliegenden Ausführungsbeispiel der Vergleichsschritt durch das Backendsystem (insbesondere eine Vergleichseinrichtung des Backendsystems) durchgeführt. Insbesondere kann das Backendsystem diesen Dienst einer Mehrzahl von mobilen Auswerte-Endgeräten (insbesondere sämtlichen im System registrierten Auswerte-Endgeräten) anbieten. Hierdurch kann unter Vermittlung eines Auswerte-Endgeräts eine Auswertung durch das Backendsystem für die in einem Ortungsgerät gespeicherten Nutzerkennungen durchgeführt werden.

Insbesondere können sämtliche in der Datenbank des Ortungsgeräts gespeicherten und durch das Ortungsgerät an das Auswerte-Endgerät übertragenen Daten (vgl. z.B. Tabelle 1) in Form eines (oder mehrerer) Nutzerkennungsdatensatzes durch das Auswerte-Endgerät an das Backendsystem übertragen werden. Dies ermöglicht es, dass in dem Vergleichsschritt sämtliche Daten berücksichtigt werden können. Die Übertragung des Nutzerkennungsdatensatzes an das Backendsystem erfolgt insbesondere über ein Fernkommunikationsnetz, vorzugsweise ein Mobilfunknetz.

Vorzugsweise kann ein Vergleichsergebnisdatensatz als Infektionsinformation durch das Backendsystem an das anfragende mobile Auswerte-Endgerät übertragen und von diesem an das entsprechende Ortungsgerät übertragen werden. Basierend auf dem Vergleichsergebnis kann ein Ausgeben einer Infektionsinformation durch das Ortungsgerät veranlasst werden, wie das Darstellen des Ergebnisses auf dem Display des Ortungsgeräts.

Anmeldungsgemäß ist erkannt worden, dass zwischen der Übertragung der mindestens einen Nutzerkennung durch das Ortungsgerät an das mobile Auswerte-Endgerät und einer Erstellung eines Vergleichsergebnisses eine bestimmte Zeitspanne vergeht. Während dieser Zeitspanne kann es vorkommen, dass sich das mobile Auswerte-Endgerät nicht mehr in Reichweite des Ortungsgeräts befindet, so dass eine Übertragung des Vergleichsergebnisdatensatzes an das Ortungsgerät über das genannte mobile Auswerte-Endgerät (derzeit) nicht möglich ist.

Um dennoch eine Übertragung des Vergleichsergebnisdatensatzes über die Nahfeldschnittstelle des Ortungsgeräts (insbesondere bei einer fehlenden Fernkommunikationsanbindung) zu ermöglichen, wird gemäß bevorzugter anmeldungsgemäßer Ausführungsformen eine asynchrone Übertragung des Vergleichsergebnisdatensatzes vorgeschlagen. Um eine asynchrone Übertragung zu gewährleisten, erfolgt, wie nachfolgend näher erläutert wird, insbesondere eine temporäre Speicherung im Backendsystem bis zur Quittierung der Übertragung an das Ortungsgerät.

Gemäß einer Ausführungsform des anmeldungsgemäßen Verfahrens kann die mindestens eine übertragene Nutzerkennung zusammen mit einer Ortungsgeräte-Kennung des Ortungsgeräts übertragen werden. Der auf der mindestens einen übertragenen Nutzerkennung basierende Nutzerkennungsdatensatz kann zumindest die Ortungsgeräte-Kennung enthalten. Insbesondere kann das mobile Auswerte-Endgerät die mindestens eine übertragene Nutzerkennung zusammen mit der zugehörigen Ortungsgeräte-Kennung in einem Nutzerkennungsdatensatz übertragen.

Dem auf dem übertragenen Nutzerkennungsdatensatz basierenden Vergleichsergebnisdatensatz kann vorzugsweise die Ortungsgeräte-Kennung (nach einer Auswertung des Nutzerkennungsdatensatzes) zugeordnet werden. Insbesondere kann das Backendsystem eine entsprechende Zuordnung vornehmen. Das Backendsystem kann vorzugsweise eine temporäre Speicherung des Vergleichsergebnisdatensatzes zusammen mit der zugeordneten Ortungsgeräte-Kennung durchführen.

Das Übertragen zumindest des Vergleichsergebnisdatensatzes an das Ortungsgerät über mindestens ein mobiles Endgerät (z.B. ein (weiteres) mobiles Auswerte-Endgerät) kann vorzugsweise auf der Ortungsgeräte-Kennung basieren, die dem Vergleichsergebnisdatensatz zugeordnet ist.

Gemäß einer bevorzugten Ausführungsform des anmeldungsgemäßen Verfahrens kann das Verfahren ferner umfassen:
- Empfangen, durch das mobile Endgerät (z.B. ein (weiteres) mobiles Auswerte-Endgerät), eines Nahfeld-Advertising-Signals des Ortungsgeräts, enthaltend zumindest die Ortungsgeräte-Kennung des Ortungsgeräts,
- Mitteilen, durch das mobile Endgerät, eines Empfangs der Ortungsgeräte-Kennung dem Backendsystem,
- Übertragen, durch das Backendsystem, des Vergleichsergebnisdatensatzes, der der mitgeteilten Ortungsgeräte-Kennung zugeordnet ist, an das mobile Endgerät, und
- zumindest teilweises Übertragen, durch das mobile Endgerät, des Vergleichsergebnisdatensatzes an das Ortungsgerät (sofern sich das mobile Endgerät noch in Reichweite des Ortungsgeräts befindet).

Insbesondere kann ein mobiles Endgerät dem Backendsystem mitteilen, dass es (derzeit) in Kontakt mit einem Ortungsgerät ist und dabei insbesondere die entsprechende Ortungsgeräte-Kennung an das Backendsystem übertragen. Das Backendsystem kann prüfen, basierend auf der Ortungsgeräte-Kennung, ob Daten für eine Übertragung an das entsprechende Ortungsgerät in dem Backendsystem gespeichert sind. Insbesondere kann gesucht werden nach einer Korrespondenz zwischen der erhaltenen Ortungsgeräte-Kennung und den jeweils einem gespeicherten Vergleichsergebnisdatensatz zugeordneten Ortungsgeräte-Kennungen. Wenn eine Korrespondenz durch das Backendsystem festgestellt wird, kann ein Übertragen des entsprechenden Vergleichsergebnisdatensatzes an das mobile Endgerät erfolgen, um insbesondere eine Übertragung des Vergleichsergebnisdatensatzes an das entsprechende Ortungsgerät zu bewirken. Sofern sich das mobile Endgerät nach Erhalt des Vergleichsergebnisdatensatzes noch immer in Reichweite der Nahfeldschnittstelle des Ortungsgeräts befindet, kann ein Übertragen des Vergleichsergebnisdatensatzes an das Ortungsgerät durchgeführt werden. Vorzugsweise kann hierfür, in zuvor beschriebener Weise, ein dauerhafter Nahfeld-Kommunikationskanal hergestellt werden.

Auf dem mobilen Endgerät kann eine entsprechende Anwendung gespeichert sein, die die vorbeschriebenen Schritte auf dem Endgerät steuert.

Bei einer bevorzugten Ausführungsform kann vorgesehen sein, dass das Backendsystem zu bestimmten Zeitpunkten (beispielsweise einmal pro Tag, einmal pro Stunde etc.) eine Broadcast-Nachricht aussendet, die eine Liste mit sämtlichen Ortungsgeräte-Kennungen umfasst, für die im Backendsystem Daten für eine Übertragung temporär gespeichert sind. Eine Broadcast-Nachricht kann von sämtlichen (registrierten) Endgeräten des Systems empfangen werden. Die Liste kann insbesondere (lokal und temporär) durch jedes Endgerät gespeichert werden. Bei einem Empfang eines vorbeschriebenen Nahfeld-Advertising-Signals eines Ortungsgeräts kann ein Mitteilen an das Backendsystem nur dann erfolgen, wenn die entsprechende Ortungsgeräte-Kennung in der (aktuellen) gespeicherten Liste enthalten ist. Andernfalls wird keine Mitteilung an das Backendsystem gesendet. Hierdurch kann die Netzbelastung und die Rechenbelastung im Backendsystem reduziert werden.

Ferner ist erkannt worden, dass ein Übertragen des Vergleichsergebnisdatensatzes durch das mobile Endgerät an das Ortungsgerät nach Erhalt des Vergleichsergebnisdatensatzes nicht erfolgreich oder nur teilweise erfolgreich sein kann. Dies meint, dass keine Daten oder nur ein Teil der in dem Vergleichsergebnisdatensatz enthaltenen Daten übertragen wurde. Der Grund hierfür kann insbesondere sein, dass sich das mobile Endgerät zwischenzeitlich nicht mehr in Reichweite der Nahbereichsschnittstelle des Ortungsgeräts befinden kann.

Gemäß einer bevorzugten Ausführungsform des anmeldungsgemäßen Verfahrens kann das Verfahren ferner umfassen:
- Aussenden, durch das mobile Endgerät, an das Backendsystem einer Bestätigungsnachricht über die vollständige Übertragung des Vergleichsergebnisdatensatzes an das Ortungsgerät,
   und/oder
- Aussenden, durch das mobile Endgerät, an das Backendsystem einer Erwiderungsnachricht über die zumindest unvollständige Übertragung des Vergleichsergebnisdatensatzes an das Ortungsgerät, wobei die Erwiderungsnachricht ein Informationsdatum über die nicht übertragenen Daten des Vergleichsergebnisdatensatzes enthält.

Hierdurch kann insbesondere sichergestellt werden, dass ein Vergleichsergebnisdatensatz tatsächlich an ein Ortungsgerät übertragen wird. Insbesondere kann vorgesehen sein, dass ein im Backendsystem gespeicherter Vergleichsergebnisdatensatz nur dann (und/oder nach Ablauf einer bestimmten Maximalspeicherdauer) gelöscht wird, wenn in dem Backendsystem eine entsprechende Bestätigungsnachricht vorliegt. Alternativ kann ein Vergleichsergebnisdatensatz in Backendsystem auch mit einer entsprechenden Information versehen werden, dass er erfolgreich übertragen wurde. Eine Löschung kann dann beispielsweise erst nach Ablauf einer bestimmten Minimalspeicherdauer erfolgen. Es versteht sich, dass nach einer Übertragung und/oder zumindest teilweise nicht erfolgreichen Übertragung die Daten des Vergleichsergebnisdatensatzes auf dem entsprechenden mobilen Endgerät wieder gelöscht werden.

Wenn die Übertragung nicht oder nur teilweise erfolgreich war, kann eine Erwiderungsnachricht ausgesendet werden, in der angegeben ist, welche Daten des Vergleichsergebnisdatensatzes nicht übertragen wurden. Es versteht sich, dass dies dadurch erfolgen kann, dass in der Erwiderungsnachricht die Daten angegebenen sind, die erfolgreich übertragen wurden. Die Erwiderungsnachricht kann vorzugsweise die Ortungsgeräte-Kennung umfassen.

Hierbei kann ein Ortungsgerät einen erfolgreichen oder teilweise erfolgreichen Empfang der Daten eines Vergleichsergebnisdatensatzes dem mobilen Endgerät quittieren.

Vorzugsweise kann das Backendsystem bei einem (im Backendsystem gespeicherten) Vergleichsergebnisdatensatz zumindest protokollieren, welche Daten des Vergleichsergebnisdatensatzes noch nicht an das zugehörige Ortungsgerät übertragen wurden. Es versteht sich, dass dies dadurch erfolgen kann, dass protokolliert wird, welche Daten erfolgreich übertragen wurden. Insbesondere bei Empfang einer Erwiderungsnachricht kann protokolliert werden, welche Daten eines entsprechenden Vergleichsergebnisdatensatzes noch zu übertragen sind.

Bei Empfang einer Mitteilung, durch das Backendsystem, von einem mobilen Endgerät über den Empfang der Ortungsgeräte-Kennung kann das Backendsystem nur die noch nicht an das Ortungsgerät übertragenen Daten des Vergleichsergebnisdatensatzes an das mobile Endgerät übertragen, wie insbesondere zuvor beschrieben wurde.

In besonders sicherer Weise kann gewährleistet werden, dass ein Vergleichsergebnisdatensatz, zumindest bei einer zuvor beschriebenen festgestellten Korrespondenz, an das Ortungsgerät (ohne eine Fernkommunikationsanbindung) übertragen wird und dass damit ein entsprechender Nutzer über den Kontakt mit einer infizierten Person tatsächlich unterrichtet werden kann.

Bei einer weiteren Ausführungsform kann das Übertragen des Nutzerkennungsdatensatzes, durch das mobile Auswerte-Endgerät, an das Backendsystem unmittelbar nach einem Empfang (sämtlicher) übertragener Nutzerkennungen erfolgen.

Gemäß einer weiteren Ausführungsform des anmeldungsgemäßen Verfahrens kann nach einem Empfang, durch das mobile Auswerte-Endgerät, der mindestens einen übertragenen Nutzerkennung und bei einer augenblicklich fehlenden Kommunikationsverbindung (insbesondere keine ausreichende Mobilfunkabdeckung) zu dem Backendsystem das mobile Auswerte-Endgerät die mindestens eine übertragene Nutzerkennung lokal zwischenspeichern. Der auf der mindestens einen zwischengespeicherten Nutzerkennung basierende Nutzerkennungsdatensatz kann, durch das mobile Auswerte-Endgerät, bei einer Detektion einer (wieder vorhandenen) Kommunikationsverbindung zu dem Backendsystem übertragen werden.

Anders ausgedrückt kann auch bei einer Übertragung von mindestens einer Nutzerkennung eines Ortungsgeräts an das Backendsystem eine asynchrone Kommunikation erfolgen. Hierdurch kann eine Übertragung an das Backendsystem stets sichergestellt werden.

Alternativ oder zusätzlich kann der Vergleichsschritt (lokal) durch das mobile Auswerte-Endgerät (insbesondere gesteuert durch eine auf dem Endgerät installierte Anwendung) erfolgen. Gemäß einer weiteren bevorzugten Ausführungsform des anmeldungsgemäßen Verfahrens kann, wie bereits beschrieben wurde, die (zentrale Infektionsdatenbank) in einem Backendsystem angeordnet sein. Bei anderen Varianten kann (bei denen keine zentrale Datenbank vorgesehen sein kann) das Backendsystem zum Verteilen von Infektionskennungen an dezentrale Datenbanken eingerichtet sein, insbesondere durch den nachfolgend beschriebenen Übertragungsschritt mindestens eines Infektionsdatensatzes.

Das Verfahren kann ferner umfassen:
- Übertragen, durch das Backendsystem, eines Infektionsdatensatzes zumindest an das mobile Auswerte-Endgerät, an das die mindestens eine Nutzerkennung übertragen wurde,
- Durchführen, durch das mobile Auswerte-Endgerät, des Vergleichsschritts, und
- Übertragen, durch das mobile Auswerte-Endgerät, zumindest eines Vergleichsergebnisdatensatzes als Infektionsinformation an das Ortungsgerät,
- wobei insbesondere bei einer augenblicklich fehlenden Kommunikationsverbindung zu dem Ortungsgerät das mobile Auswerte-Endgerät den Vergleichsergebnisdatensatz lokal zwischenspeichert und/oder an das Backendsystem überträgt.

Der mindestens eine Infektionsdatensatz kann zumindest die gespeicherten Infektionskennungen enthalten. Der Vorteil von dezentralen Auswertungen ist, dass die Rechenbelastung des Backendsystems reduziert werden kann. Das Backendsystem hat nur die Aufgabe, Infektionskennungen weiterzuleiten bzw. zu verteilen und/oder zu speichern (wie zuvor beschrieben wurde) und die gespeicherten Daten an die mobilen Auswerte-Endgeräte zu übertragen. Zusätzlich kann das Backendsystem die Aufgabe haben, Vergleichsergebnisdatensätze temporär zu speichern sowie eine Übertragung an ein entsprechendes Ortungsgerät zu veranlassen, wie zuvor beschrieben wurde.

Der Vergleichsschritt wird bei dieser Ausführungsform dezentral von den entsprechenden mobilen Auswerte-Endgeräten durchgeführt.

Vorzugsweise kann vorgesehen sein, dass nur Infektionskennungen (und insbesondere die jeweils zugehörigen Infektionsangaben) ausgesendet werden, die innerhalb eines bestimmten Zeitbereichs liegen, der insbesondere von der Inkubationszeit einer Infektionskrankheit abhängen kann (z.B. sämtliche Infektionskennungen der vorherigen x Tage).

Darüber hinaus kann das Übertragen zu bestimmten Zeitpunkten erfolgen, beispielsweise regelmäßig, und/oder bei Überschreiten einer bestimmten Infektionskennungsanzahl, also wenn beispielsweise (in einem Zeitraum X) über Y (entspricht der bestimmten Infektionskennungsanzahl) Infektionskennungen gespeichert wurden. Auch kann das Aussenden auf Anfrage eines mobilen Auswerte-Endgeräts erfolgen. Insbesondere kann ein Auswerte-Endgerät eine derartige Anfrage an das Backendsystem insbesondere unmittelbar nach einer Übertragung der mindestens einen in dem Ortungsgerät gespeicherten Nutzerkennung an das Auswerte-Endgerät senden.

Bevorzugt kann ein Übertragen des Infektionsdatensatzes an sämtliche im System registrierte mobile Auswerte-Endgeräte erfolgen. Insbesondere kann ein Aussenden eines Broadcast-Nachricht erfolgen, enthaltend den Infektionsdatensatz, wobei nur ein mobiles Auswerte-Endgerät den Infektionsdatensatz verarbeiten kann, auf dem eine Anwendung installiert ist. Dies kann zu einer Reduzierung der Netzlast des verwendeten Kommunikationsnetzes (insbesondere ein Mobilfunknetz) führen.

Gemäß einer weiteren Ausführungsform kann das Übertragen eines Infektionsdatensatzes verschlüsselt erfolgen. Alternativ oder vorzugsweise zusätzlich kann das Übertragen eines Nutzerkennungsdatensatzes verschlüsselt erfolgen. Die Datensicherheit kann noch weiter verbessert werden.

Auch bei einer lokalen Auswertung kann durch eine asynchrone Kommunikation sichergestellt werden, dass der resultierende Vergleichsergebnisdatensatz an das entsprechende Ortungsgerät übertragen wird.

Bei einer augenblicklich fehlenden Kommunikationsverbindung zu dem Ortungsgerät kann das mobile Auswerte-Endgerät den Vergleichsergebnisdatensatz lokal zwischenspeichern und/oder an das Backendsystem (für eine Zwischenspeicherung) übertragen. Bei der zuletzt genannten Option kann die Übertragung an das Ortungsgerät erfolgen, wie zuvor beschrieben wurde. Ein zwischengespeicherter Vergleichsergebnisdatensatz kann bei einer Detektion einer (erneuten) Kommunikationsverbindung zu dem Ortungsgerät übertragen werden.

Gemäß einer besonders bevorzugten Ausführungsform des anmeldungsgemäßen Verfahrens können in dem Vergleichsschritt zumindest die Infektionszeitangabe, die Empfangszeitpunktangabe und die Infektionskennung (insbesondere die Inkubationszeit der entsprechenden Infektionskrankheit) berücksichtigt werden. Insbesondere können in dem Vergleichsschritt aufgrund der verschiedenen Zeitangaben die Kennungen unberücksichtigt bleiben, bei denen aufgrund der Zeitangaben eine Ansteckung ausgeschlossen werden kann. Beispielsweise werden von dem mobilen Auswerte-Endgerät nur Kennungen (und ggf. zugehörige Angaben) berücksichtigt, welche zeitlich in einem bestimmten Zeit-Intervall vor dem Datum (insbesondere dem Datum, an dem die Krankheit nachgewiesen wurde) in der (zentralen) Infektionsdatenbank liegen (Inkubationszeit + ggfs. Dauer der Erkrankung).

Gemäß einer weiteren bevorzugten Ausführungsform des anmeldungsgemäßen Verfahrens kann das Verfahren ferner umfassen:
- Aussenden, durch das Ortungsgerät, eines Nahfeld-Signals, enthaltend die Ortsgeräte-Kennung und ein Vergleichsergebnisdatum,
- wobei das Vergleichsergebnisdatum insbesondere angibt, ob ein Kontakt zu einer infizierten Person bestand (oder nicht).

Beispielsweise gesteuert durch eine auf dem Ortungsgerät installierte Anwendung kann ein (nahezu kontinuierliches bzw. regelmäßiges) Aussenden eines (zuvor beschriebenen) Nahfeld-(Advertising-)Signals erfolgen. Das Nahfeld-Signal, vorzugsweise ein zuvor beschriebenes Advertising-Signal, kann neben der Ortungsgeräte-Kennung ein Vergleichsergebnisdatum (und ggf. ein Auswerteinformationsdatum) enthalten.

Das Vergleichsergebnisdatum hängt insbesondere von dem Vergleichsergebnis eines Vergleichsschritts ab, also ob beispielsweise eine zuvor beschriebene Korrespondenz festgestellt worden ist oder nicht. Anders ausgedrückt gibt das Vergleichsergebnisdatum insbesondere an, ob ein Kontakt zu einer infizierten Person bestand (oder nicht). Als Vergleichsergebnisdatum kann beispielsweise ein Flag in dem Nahfeld-Signal bzw. der Nahfeld-Nachricht gesetzt (z.B. um anzuzeigen, dass es einen Kontakt mit einer infizierten Person gegeben hat) oder nicht gesetzt werden (z.B. um anzuzeigen, dass es keinen Kontakt mit einer infizierten Person gegeben hat).

Wenn ein Nahfeld-Signal von einem mobilen Endgerät oder einem weiteren Ortungsgerät empfangen wird, welches ein Vergleichsergebnisdatum enthält, kann dies in der entsprechenden Datenbank abgespeichert werden, zusammen mit der entsprechenden Nutzerkennung. Auch kann ein (unmittelbares) Ausgeben einer entsprechenden Infektionsinformation bei Erhalt eines entsprechenden Nahfeld-Signals erfolgen. Hierdurch kann der Nutzer des empfangenen mobilen Endgeräts oder Ortungsgeräts zeitnah informiert werden.

Gemäß einer weiteren Ausführungsform des anmeldungsgemäßen Verfahrens kann das Verfahren ferner umfassen:
- Registrieren eines Ortungsgeräts in einem zentralen Register durch Speichern zumindest der Ortungsgeräte-Kennung des Ortungsgeräts in dem zentralen Register,
- wobei das Registrieren ein Ausgeben, durch das Ortungsgerät, der Ortungsgeräte-Kennung umfasst, und
- wobei das Registrieren ein Übertragen der ausgegebenen Ortungsgeräte-Kennung durch ein weiteres Gerät an das zentrale Register umfasst.

Insbesondere kann ein neues Ortungsgerät in einfacher Weise (auch ohne eigene Fernkommunikationsanbindung) in einem zentralen Register registriert werden. Das zentrale Register kann insbesondere in dem vorbeschriebenen Backendsystem implementiert sein.

Das Ausgeben der Ortungsgeräte-Kennung kann beispielsweise über ein Display des Ortungsgeräts erfolgen. Die Ortungsgeräte-Kennung kann (z.B. nach einer Detektion eines Drückens eines Tasters oder dergleichen) auf dem Display (z.B. als QR-Code oder dergleichen) angezeigt und beispielsweise durch ein mobiles Endgerät (z.B. Smartphone) eingescannt werden.

Alternativ oder zusätzlich kann das Ausgeben ein Übertragen der Ortungsgeräte-Kennung über die Nahfeldschnittstelle des Ortungsgeräts erfolgen. Insbesondere unter Steuerung einer Anwendung des mobilen Endgeräts kann eine Registrierung des Ortungsgeräts erfolgen. Über die Anwendung können zusätzliche Informationen mitgegeben werden, z.B. Notfall- Kontaktdaten (z.B. E-Mail, Telefonnummer), Geopositionsdaten (insbesondere bei einem stationären Ortungsgerät) etc. Diese Daten können zumindest teilweise einem zuvor beschriebenen Vergleichsergebnisdatensatz hinzugefügt werden.

Alternativ kann ein Webinterface für eine Registrierung genutzt werden, bei dem die Kennung des Ortungsgeräts eingegeben werden kann.

Wie bereits beschrieben wurde, kann das Ortungsgerät einem Nutzer (systemweit eineindeutig) zugeordnet sein. Gemäß einer weiteren Ausführungsform des anmeldungsgemäßen Verfahrens kann bei einer Infektion des Nutzers des Ortungsgeräts ein verifiziertes Endgerät die Ortungsgeräte-Kennung der zentralen Infektionsdatenbank als verifizierte Infektionskennung melden.

Bei dieser Ausführungsform erfolgt insbesondere eine Fremdmeldung über eine autorisierte Instanz: Eine entsprechende autorisierte Nutzerkennung eines verifizierten Endgeräts (z.B. eines Arztes oder dergleichen) kann die Ortungsgeräte-Kennung als Infektionskennung melden. Entsprechende Kontakte der infizierten Person können im Meldeprozess mitübertragen werden (z.B. per Advertising-Signal oder codiert im QR-Code via QR-Scan). Alternativ kann die Infektionsmeldung in einer (zentralen) Infektionsdatenbank über ein Webinterface von autorisierten Personen erfolgen. Wie beschrieben wurde, kann bei dezentralen Datenbanken das Backendsystem eine Verteilung der Infektionsmeldungen und/oder Infektionskennungen an diese Datenbanken (für eine entsprechende Aktualisierung) steuern. Die Kontaktdaten können dann über eine Anfrage des Backendsystems über ("fremde") mobile Endgeräte/APP "eingesammelt" werden. Infektionsmeldungen über eine autorisierte Instanz könnten mit einer Klassifikation versehen werden z.B. "Confirmed" (oder dergleichen).

Alternativ oder zusätzlich kann bei einer Infektion des Nutzers ein nicht-verifiziertes Endgerät die Ortungsgeräte-Kennung der (zentralen) Infektionsdatenbank als nicht-verifizierte Infektionskennung melden, wobei insbesondere durch ein verifiziertes Endgerät eine nicht-verifizierte Infektionskennung verifiziert werden kann.

Insbesondere kann eine Selbstmeldung erfolgen, vorzugsweise ebenfalls über ein mobiles Endgerät bzw. über die darauf installierte Anwendung oder über ein Webinterface, wie oben beschrieben. Zur Unterscheidung von der zuvor beschriebenen "Fremdmeldung" kann diese Meldung mit einer Klassifikation "Selftest" (oder dergleichen) versehen werden. Dies wiederum kann zu einer zusätzlich notwendigen Überprüfung der Meldung durch eine verifizierte Instanz (z.B. Arzt) bzw. ein verifiziertes Endgerät einer solchen Instanz führen. Die Information über die Klassifikation "Selftest" (oder dergleichen) kann an Personen, die mit dieser Person Kontakt hatten, weitergeleitet werden. Ggf. können weitere Mitteilungsmechanismen variiert werden.

Gemäß einer bevorzugten Ausführungsform des anmeldungsgemäßen Verfahrens kann das Ortungsgerät ein stationäres Ortungsgerät sein, das einer bestimmten Entität (dauerhaft) zugeordnet sein kann. Die Entität ist insbesondere ein Gebäude oder ein Fahrzeug (und insbesondere keine Person).

Vorzugsweise kann an mindestens einer Zugangsordnung und/oder mindestens einem Eingang/Ausgang eines Gebäudes (z.B. Bahnhof, Krankenhaus, Bürogebäude, Pflegeheim) und/oder eines Fahrzeugs (z.B. Bus, Schienenfahrzeug etc.) ein Ortungsgerät fest installiert sein. Die Installationsposition des Ortungsgeräts kann bei der Registrierung in dem zentralen Register gespeichert werden und/oder in dem Ortungsgerät hinterlegt werden. Durch die Montierung von Ortungsgräten insbesondere an Ein- und/oder Ausgängen können vorzugsweise sämtliche Personen erfasst werden, die ein bestimmtes Gebäude und/oder ein bestimmtes Fahrzeug betreten und/oder verlassen. Bei einem stationären Ortungsgerät können die vorbeschriebene Speicherkriterien vorzugsweise nicht angewendet werden.

Ein stationäres Ortungsgerät kann eine spezifische Ortungsgeräte-Kennung enthalten, die das Ortungsgerät als stationäres Ortungsgerät kennzeichnet. Beim Kontakttracking bzw. bei der Kontaktauswertung können für diese spezifischen Ortungsgeräte-Kennungen besondere Verfahren definiert werden. Z.B. können für diese spezifischen Ortungsgeräte-Kennungen Speicherkriterien aufgehoben werden. Dies kann insbesondere in Hochrisikobereichen zur besseren Rückverfolgbarkeit von Infektionsketten führen.

Es versteht sich, dass ein Ortungsgerät auch an anderen Positionen eines Gebäudes und/oder eines Fahrzeugs stationär installiert sein kann.

Ein weiterer Aspekt der Anmeldung ist ein Ortungsgerät, umfassend:
- mindestens eine Nahfeldschnittstelle, eingerichtet zum Empfangen mindestens eines Nahfeld-Signals mindestens eines in Empfangsreichweite des Ortungsgeräts befindlichen mobilen Endgeräts oder eines weiteren Ortungsgeräts,
- mindestens eine Datenbank, eingerichtet zum Speichern mindestens einer Nutzerkennung, wobei die Nutzerkennung in dem mindestens einen empfangenen Nahfeld-Signal enthalten ist,
- wobei die Nahfeldschnittstelle eingerichtet ist zum Aussenden eines Nahfeld-Advertising-Signals, enthaltend zumindest ein Auswerteinformationsdatum, das zumindest eine potentielle Notwendigkeit einer Auswertung der mindestens einen gespeicherten Nutzerkennung angibt, wobei die Auswertung zumindest ein Vergleichen der mindestens einen in der Datenbank des Ortungsgeräts gespeicherten Nutzerkennung mit mindestens einer in einer (zentralen) Infektionsdatenbank gespeicherten Infektionskennung umfasst, die einem eine Infektionskrankheit aufweisenden Nutzer zugeordnet ist,
- wobei die Nahfeldschnittstelle eingerichtet ist zum Empfangen eines Antwortsignals auf das ausgesendete Nahfeld-Advertising-Signal von einem mobilen Auswerte-Endgerät, wobei das Antwortsignal ein Antwortdatum enthält, das zumindest eine Bereitschaft des mobilen Auswerte-Endgeräts zum Veranlassen der Auswertung der mindestens einen gespeicherten Nutzerkennung angibt, und
- wobei die Nahfeldschnittstelle eingerichtet ist zum Übertragen der mindestens einen gespeicherten Nutzerkennung an das mobile Auswerte-Endgerät zur Auswertung der mindestens einen gespeicherten Nutzerkennung, wenn das Ortungsgerät das Antwortsignal erhält und die Auswertung notwendig ist..

Das vorbeschriebene Verfahren kann insbesondere zur zumindest teilweisen Steuerung des Ortungsgeräts verwendet werden. Auf dem Ortungsgerät kann insbesondere eine Anwendung installiert bzw. implementiert sein, die die vorgenannten Schritte des Verfahrens ausführt und insbesondere steuert.

Ein noch weiterer Aspekt ist ein System, umfassend mindestens ein zuvor beschriebenes Ortungsgerät.

Das vorbeschriebene Verfahren kann insbesondere zum Betreiben des Systems verwendet werden. Das System dient zum Identifizieren eines Nutzers eines Ortungsgeräts, der sich temporär in einer räumlichen Distanz zu einem infizierten Nutzer befunden hat.

Wie bereits beschrieben wurde, kann die zentrale Infektionsdatenbank in einem Backendsystem angeordnet sein und insbesondere von diesem gesteuert werden. Das System kann insbesondere das Backendsystem umfassen. Ein anmeldungsgemäßes Backendsystem kann umfassen:
- mindestens eine zentrale Infektionsdatenbank, enthaltend mindestens eine gespeicherte Infektionskennung, die einem eine Infektionskrankheit aufweisenden Nutzer zugeordnet ist, und
- mindestens eine Vergleichseinrichtung, eingerichtet zum Vergleichen der mindestens einen gespeicherten Infektionskennung mit mindestens einer Nutzerkennung, die in einer Datenbank eines (zuvor beschriebenen) Ortungsgerät gespeichert ist, wobei das Ortungsgerät die mindestens eine gespeicherte Nutzerkennung von mindestens einem mobilen Endgerät oder einem weiteren Ortungsgerät durch ein Nahfeld-Signal empfangen hat.

Insbesondere kann das Backendsystem (z.B. gebildet durch einen oder mehrere verteilt in einer Cloud angeordneten Server) zur (zumindest teilweisen) Durchführung des zuvor beschriebenen Verfahrens verwendet werden.

Das System kann mindestens eine Vergleichseinheit umfassen. Die mindestens eine Vergleichseinheit kann in dem Backendsystem (z.B. in Form einer Vergleichseinrichtung) implementiert sein und/oder auf dem mobilen Auswerte-Endgerät (z.B. in Form eines Vergleichsmoduls einer auf dem mobilen Auswerte-Endgerät installierten Anwendung).

Ferner kann das System mindestens eine Detektionseinheit umfassen, eingerichtet zum Detektieren einer Korrespondenz zwischen der mindestens einen gespeicherten Nutzerkennung und der mindestens einen gespeicherten Infektionskennung. Die mindestens eine Detektionseinheit kann in dem Backendsystem implementiert sein und/oder auf dem mobilen Auswerte-Endgerät (z.B. in Form eines Detektionsmoduls einer auf dem mobilen Auswerte-Endgerät installierten Anwendung). Die Detektionseinheit kann insbesondere in der Vergleichseinheit integriert sein.

Darüber hinaus kann das System mindestens eine Ausgabeeinheit umfassen, eingerichtet zum Ausgeben einer Infektionsinformation, zumindest bei einer Detektion einer Korrespondenz zwischen der mindestens einen gespeicherten Nutzerkennung und der mindestens einen gespeicherten Infektionskennung. Die mindestens eine Ausgabeeinheit kann in dem Backendsystem (z.B. in Form einer Kommunikationseinrichtung) implementiert sein und/oder auf dem Ortungsgerät (z.B. in Form eines Ausgabemoduls einer auf dem Ortungsgerät installierten Anwendung).

Ein noch weiterer Aspekt der Anmeldung ist eine Anwendung zum Rekonstruieren von Infektionsketten, insbesondere in Form einer durch einen Prozessor eines mobilen Auswerte-Endgeräts ausführbaren Softwareanwendung, zur Installation auf dem mobilen Auswerte-Endgerät. Die Anwendung umfasst:
- mindestens ein Empfangsmodul, eingerichtet zum Erhalten mindestens einer Nutzerkennung von einem zuvor beschriebenen Ortungsgerät,
- mindestens ein Detektionsmodul, eingerichtet zum Detektieren, in einem Vergleichsschritt, dass die mindestens eine erhaltene Nutzerkennung des Ortungsgerät korrespondiert mit mindestens einer in einer (zentralen) Infektionsdatenbank gespeicherten Infektionskennung, die einem eine Infektionskrankheit aufweisenden Nutzer zugeordnet ist, und
- mindestens ein Ausgabemodul, eingerichtet zum Bewirken eines Ausgebens einer Infektionsinformation, zumindest wenn in dem Vergleichsschritt mindestens ein Korrespondieren detektiert wird.

Insbesondere kann die (Auswerte-) Anwendung zur (zumindest teilweisen) Durchführung des zuvor beschriebenen Verfahrens verwendet werden.

Die Anwendung ist insbesondere eine auf einem mobilen Auswerte-Endgerät installierbare Softwareanwendung. Durch Installation auf dem mobilen Auswerte-Endgerät wird eine zufällige Kennung generiert. Diese Kennung dient später als Identifizierung zur Versendung von Mitteilungen etc. Die mit dem Nahfeld-Signal ausgesendeten Nutzerkennungen (IDs) können zeitlich variieren und brauchen nicht mit der Kennung identisch zu sein, müssen dieser aber zuordenbar sein, zum Beispiel durch ein ID-Logging im mobilen Auswerte-Endgerät.

Das mindestens eine Empfangsmodul der Anwendung kann ferner eingerichtet sein zum Erhalten mindestens einer in einem Nahfeld-Signal enthaltenen Nutzerkennung, wobei das Nahfeld-Signal durch das mobile Auswerte-Endgerät empfangen wurde,

Die Anwendung kann ferner umfassen:
- mindestens ein Speichermodul, eingerichtet zum Speichern der erhaltenen Nutzerkennung in einer (dem mobilen Auswerte-Endgerät zugeordneten) Datenbank,
- wobei das Detektionsmodul eingerichtet ist zum Detektieren, in einem Vergleichsschritt, dass die mindestens eine in der Datenbank gespeicherte Nutzerkennung korrespondiert mit mindestens einer in einer (zentralen) Infektionsdatenbank gespeicherten Infektionskennung, die einem eine Infektionskrankheit aufweisenden Nutzer zugeordnet ist, und
- wobei das Ausgabemodul eingerichtet ist zum Bewirken eines Ausgebens einer Infektionsinformation, zumindest wenn in dem Vergleichsschritt mindestens ein Korrespondieren detektiert wird.

Die Anwendung in Form eines Computerprogramms, insbesondere die Instruktionen bzw. Programmanweisungen, können in einem Computerprogrammprodukt gespeichert sein, insbesondere einem Programmspeicher. Zum Beispiel ist ein Programmspeicher ein nicht-flüchtiger Speicher wie ein Flash-Speicher, ein Magnetspeicher, ein EEPROM-Speicher (elektrisch löschbarer programmierbarer Nur-Lese-Speicher) und/oder ein optischer Speicher.

Zusätzlich kann ein mobiles Auswerte-Endgerät einen Hauptspeicher aufweisen, zum Beispiel einen flüchtigen oder nicht-flüchtigen Speicher, insbesondere einen Speicher mit wahlfreiem-Zugriff (RAM), wie ein statischer RAM-Speicher (SRAM), ein dynamischer RAM-Speicher (DRAM), ein ferroelektrischer RAM-Speicher (FeRAM) und/oder ein magnetischer RAM-Speicher (MRAM). Der Prozessor des mobilen Endgeräts kann beispielsweise Zwischenergebnisse oder Ähnliches in dem Hauptspeicher speichern.

Gemäß einer bevorzugten Ausführungsform der Anwendung kann die Anwendung mindestens ein Vergleichsmodul (als Vergleichseinheit) umfassen, eingerichtet zum Durchführen des Vergleichsschritts, insbesondere des zuvor beschriebenen und von dem mobilen Auswerte-Endgerät durchgeführten Vergleichsschritts.

Ein weiterer Aspekt der Anmeldung ist ein mobiles Auswerte-Endgerät, umfassend eine auf dem mobilen Endgerät installierte und zuvor beschriebene Anwendung.

Bei anderen Varianten der Anmeldung kann ein Ortungsgerät auch zusätzlich über eine Fernkommunikationsschnittstelle verfügen, jedoch ohne eine Telefoniefunktionalität. Beispielsweise kann das Ortungsgerät über eine (reichweiten) beschränkte Fernkommunikationsschnittstelle verfügen, die beispielsweise basiert auf NB (Narrow-Band)-IoT.

Ein zuvor beschriebenes Modul, eine Einrichtung, eine Einheit etc. kann zumindest teilweise Hardwareelemente (z.B. Prozessor, Speichermittel etc.) und/oder zumindest teilweise Softwareelemente (z.B. ausführbaren Code) umfassen.

Die Merkmale der Verfahren, Systeme, Anwendungen, Ortungsgeräte und mobilen Auswerte-Endgeräte sind frei miteinander kombinierbar. Insbesondere können Merkmale der Beschreibung und/oder der abhängigen Ansprüche, auch unter vollständiger oder teilweiser Umgehung von Merkmalen der unabhängigen Ansprüche, in Alleinstellung oder frei miteinander kombiniert eigenständig erfinderisch sein.

Es gibt nun eine Vielzahl von Möglichkeiten, das anmeldungsgemäße Verfahren, das anmeldungsgemäße System, die anmeldungsgemäße Anwendung, das anmeldungsgemäße Ortungsgerät und das anmeldungsgemäße mobile Auswerte-Endgerät auszugestalten und weiterzuentwickeln. Hierzu sei einerseits verwiesen auf die den unabhängigen Patentansprüchen nachgeordneten Patentansprüche, andererseits auf die Beschreibung von Ausführungsbeispielen in Verbindung mit den Zeichnungen. In den Zeichnungen zeigt:
- Fig. 1: eine schematische Ansicht eines Ausführungsbeispiels eines Systems gemäß der vorliegenden Anmeldung
- Fig. 2: ein Diagramm eines Ausführungsbeispiels eines Verfahrens gemäß der vorliegenden Anmeldung.
- Fig. 3: eine schematische Ansicht eines weiteren Ausführungsbeispiels eines Systems gemäß der vorliegenden Anmeldung mit einem Ausführungsbeispiel einer Anwendung gemäß der vorliegenden Anmeldung,
- Fig. 4: eine schematische Ansicht eines weiteren Ausführungsbeispiels eines Systems gemäß der vorliegenden Anmeldung mit einem weiteren Ausführungsbeispiel einer Anwendung,
- Fig. 5: eine schematische Ansicht eines Ausführungsbeispiels eines Systems gemäß der vorliegenden Anmeldung, und
- Fig. 6: eine schematische Ansicht eines Ausführungsbeispiels eines Systems gemäß der vorliegenden Anmeldung.

Nachfolgend werden für gleiche Elemente gleiche Bezugszeichen verwendet.

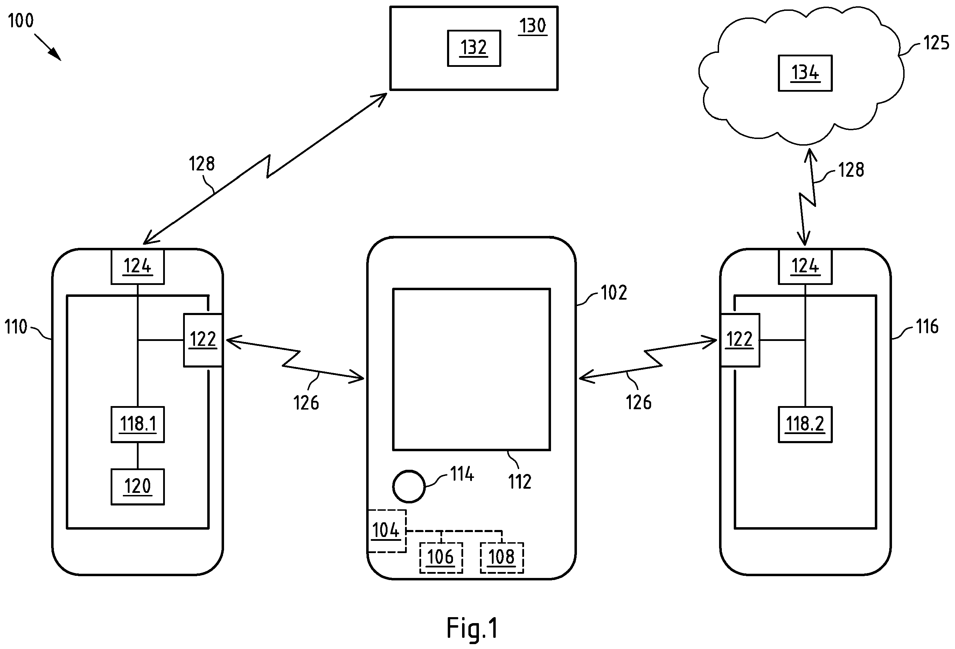

Die Figur 1 zeigt eine schematische Ansicht eines Ausführungsbeispiels eines Systems 100 gemäß der vorliegenden Anmeldung mit einem Ausführungsbeispiel eines Ortungsgeräts 102 gemäß der vorliegenden Anmeldung. Insbesondere kann das System 100 gemäß dem anmeldungsgemäßen Verfahren betrieben werden.

Das System 100 umfasst mindestens ein Ortungsgerät 102. Das Ortungsgerät 102 weist eine begrenzte Funktionalität auf. Vorliegend weist das Ortungsgerät 102 ein Display 112, einen von einem Nutzer betätigbaren Taster 114, eine (einzelne) Kommunikationsschnittstelle 104 in Form einer Nahbereichsschnittstelle 104, eine Datenbank 106 und eine auf dem Ortungsgerät 102 installierte und von einem nicht gezeigten Prozessor ausführbare Anwendung 108 auf (insbesondere eine Softwareanwendung 108).

Beispielhaft kann ein Ortungsgerät 102 folgende Hardware-Eigenschaften besitzen:
- Drahtlose Kommunikationsschnittstelle 104 [Sende/Empfänger] (Bluetooth, WLAN)
- Bluetooth-Flash-Speicher (256 kB, optional zusätzlich SPI-Flash)
- Optional: Vibrationssensor
- Optional: Beschleunigungssensor
- Optional: Vibrationsmotor
- Display 112
- Taster 114
- Optional: 2 LED
- Batterie (z.B. Knopfzelle)

Beispielhaft kann ein Ortungsgerät 102 (insbesondere die Anwendung 108) folgende Software-Eigenschaften besitzen:
- Eindeutige Ortungsgeräte-Kennung (z.B. Mac-Adresse etc.)
- Die Ortungsgeräte-Kennung kann über Display 112 in Form eines QR-Code angezeigt werden
- Die Ortungsgeräte-Kennung kann via drahtloser Kommunikationsschnittstelle 114 ausgesendet werden (z.B. BLE-Advertising-Signal)
- Der Taster 114 kann u.a.: Display 112 aktivieren; Anzeigen auf dem Display 112 steuern; Inhalte, Art und Frequenzen der angezeigten Daten (Display 112) und/oder ausgesendeten Daten (Kommunikationsschnittstellen 104) variieren.

Ferner kann das System 100 mindestens ein mobiles Endgerät 116 umfassen. Auf dem mobilen Endgerät 116 kann eine Anwendung 118.2 installiert sein. Dem mobilen Endgerät 116 ist eine Datenbank 134 zugeordnet. Die dezentrale Datenbank 134 ist vorliegend in einer Cloud 125 angeordnet. Über ein (Fern-) Kommunikationsnetz 128 können Daten, insbesondere Nutzerkennungen, in der Datenbank 134 gespeichert werden. Es versteht sich, dass in der Cloud (und/oder mindestens einer weiteren Cloud) eine Mehrzahl von Datenbanken für eine entsprechende Mehrzahl von mobilen Endgeräten angeordnet sein kann. Es versteht sich weiter, dass die Datenbank 134, die dem mobilen Endgerät 116 zugeordnet ist, auch lokal in dem mobilen Endgerät 116 angelegt sein kann.

Das mobile Endgerät 116 weist mindestens eine Nahfeldschnittstelle 122 und mindestens eine Fernkommunikationsschnittstelle 124 auf. Das Ortungsgerät 102 verfügt über keine Fernkommunikationsschnittstelle.

Ferner ist mindestens ein mobiles Auswerte-Endgerät 110 vorgesehen, welches ähnlich wie das mobile Endgerät 116 gebildet sein kann. Ein Auswerte-Endgerät 110 ist ein mobiles Endgerät 110, das eine Auswertung selbst durchführen kann oder eine Auswertung durch ein Backendsystem 130 unmittelbar veranlassen kann.

Das mobile Auswerte-Endgerät 110 weist mindestens eine Nahfeldschnittstelle 122 und mindestens eine Fernkommunikationsschnittstelle 124 auf. Darüber hinaus weist das mobile Auswerte-Endgerät 110 mindestens eine (Auswerte-)Anwendung 118.1 und eine lokale Datenbank 120 auf, um insbesondere Daten zwischenzuspeichern. Bei weiteren Varianten kann das mobile Auswerte-Endgerät 110 auch eine lokale Datenbank entsprechend der Datenbank 134 aufweisen, zum Speichern von empfangenen Nutzerkennungen.

Darüber hinaus umfasst das System 100 in dem vorliegenden Ausführungsbeispiel ein Backendsystem 130. In dem Backendsystem 130 ist in dem vorliegenden Ausführungsbeispiel eine zentrale Infektionsdatenbank 132 implementiert.

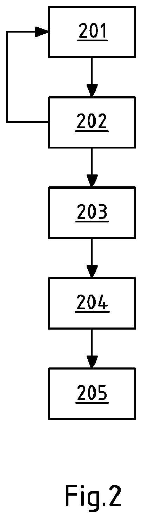

Die Funktionsweise wird nachfolgend näher mit der Figur 2 beschrieben. Die Figur 2 zeigt ein Diagramm eines Ausführungsbeispiels eines Verfahrens gemäß der vorliegenden Anmeldung.

In einem optionalen (nicht gezeigten) Schritt kann zunächst eine Registrierung des Ortungsgeräts 102 erfolgen, insbesondere durch Speichern zumindest der Ortungsgeräte-Kennung des Ortungsgeräts 102 in einem (nicht gezeigten) zentralen Register (z.B. des Backendsystems 130), wobei das Registrieren ein Ausgeben, durch das Ortungsgerät 102, der Ortungsgeräte-Kennung umfasst, und wobei das Registrieren ein Übertragen der ausgegebenen Ortungsgeräte-Kennung durch ein weiteres Gerät 110, 116 an das zentrale Register umfasst.

In einem Schritt 201 erfolgt ein Empfangen, durch das Ortungsgerät 102, mindestens eines Nahfeld-Signals 126 mindestens eines in Empfangsreichweite des Ortungsgeräts 102 befindlichen mobilen Endgeräts 110, 116 (oder eines weiteren nicht dargestellten Ortungsgeräts). Das Ortungsgerät 102 kann aus dem Nahfeld-Signal 126 die Nutzerkennung extrahieren und insbesondere ein Speichern mindestens einer Nutzerkennung in der Datenbank 106 veranlassen.

Dies kann vorzugsweise durch die Anwendung 108 gesteuert werden. Die mindestens eine Nahfeldschnittstelle 122 eines mobilen Endgeräts 110, 116 kann vorzugsweise eine Bluetooth-Schnittstelle 122 sein. Bei einer aktivierten Bluetooth-Schnittstelle 122 kann ein mobiles Endgerät 110, 116 (nahezu kontinuierlich) Nahfeld-Signale 126 in Form von Bluetooth-Advertising-Signalen 126 bzw. -Nachrichten 126 aussenden, um die Anwesenheit und die grundsätzliche Bereitschaft eines mobilen Endgeräts 110, 116 für eine Kopplung einem weiteren (in Reichweite befindlichem) mobilen Endgerät 110, 116 oder einem Ortungsgerät 102 anzuzeigen.

Jedes Nahfeld-Signal 126 kann insbesondere zumindest eine Nutzerkennung des aussendenden mobilen Endgeräts 110, 116 enthalten. In entsprechender Weise kann auch das Ortungsgerät 102 (nahezu kontinuierlich) Nahfeld-Signale 126 in Form von Bluetooth-Advertising-Signalen 126 bzw. -Nachrichten 126 aussenden.

In Schritt 202 erfolgt ein Speichern mindestens einer Nutzerkennung in der Datenbank 106 des Ortungsgeräts 102, wobei die Nutzerkennung in dem mindestens einen, durch das Ortungsgerät102 empfangenen Nahfeld-Signal 126 enthalten ist.

Wie bereits beschrieben wurde, kann aus jedem empfangenen (vorliegend) Bluetooth-Signal 126 die enthaltene Nutzerkennung des aussendenden mobilen Endgeräts 110, 116 extrahiert werden. Ein (nicht gezeigtes) Empfangsmodul der Anwendung 108 kann eingerichtet sein zum Erhalten der Nutzerkennung von der Bluetooth-Nahfeldschnittstelle 104. Das Empfangsmodul kann weitere Angaben erhalten, wie den RSSI des empfangenen Bluetooth-Signals (oder eine bereits hieraus bestimmte Distanz zu dem sendenden mobilen Endgerät), den Empfangszeitpunkt des Bluetooth-Signals etc. Die Distanz kann beispielsweise durch ein (nicht dargestelltes) RSSI-Modul des Ortungsgeräts 102 und/oder der Anwendung 108 in (grundsätzlich) bekannter Weise bestimmt werden.

Das Empfangsmodul kann die empfangenen Angaben (zumindest die Nutzerkennung) einem (nicht gezeigten) Speichermodul der Anwendung 108 bereitstellen. Das Speichermodul kann eingerichtet sein zumindest zum Speichern der erhaltenen Nutzerkennung in der Datenbank 106.

Vor einer (finalen) Speicherung kann das Speichermodul die erhaltenen Angaben auswerten. Beispielsweise können (nahezu kontinuierlich bzw. wiederholend) Bluetooth-Signale 126 mit der gleichen Nutzerkennung von dem gleichen sendenden mobilen Endgerät 110, 116 empfangen werden. Das Speichermodul kann insbesondere die Empfangsdauer bestimmen, während der die Bluetooth-Signale mit gleicher Nutzerkennung empfangen werden. Anders ausgedrückt, kann eine (zuvor beschriebene) Zeitdauerangabe bestimmt werden.

Bei einer Mehrzahl von Bluetooth-Signalen 126 mit gleicher Nutzerkennung kann zudem zur Bestimmung der Distanzangabe eine Minimumbestimmung erfolgen, um insbesondere die geringste Distanz während der Empfangsdauer als Distanzangabe zu bestimmen. Zusammen mit der jeweiligen Nutzerkennung können dann die zugehörigen Angaben in der Datenbank 106 gespeichert werden (vgl. z.B. Tabelle 1).

Besonders bevorzugt kann zusätzlich eine Geopositionsangabe gespeichert werden, über die geographische Position des sendenden mobilen Endgeräts 110, 116, wenn eine entsprechende Angabe in dem Nahfeld-Signal 126 enthalten ist.

Bei anderen Varianten kann auch die geographische Position des Ortungsgeräts 102 bestimmt werden (wenn das Ortungsgerät 102 über entsprechende Fähigkeiten verfügt). Beispielsweise kann ein Nutzer in der Anwendung 108 ein Erfassen und insbesondere ein Aufzeichnen seiner Geoposition bei Empfang eines Nahfeld-Signals 126 freigegeben haben. Vorzugsweise können dem Empfangsmodul (bei Empfang eines Nahfeld-Signals) augenblickliche GPS-Koordinaten eines (nicht dargestellten) GPS-Sensors des Ortungsgeräts 102 bereitgestellt werden. Das Speichermodul kann dann eine Speicherung der Nutzerkennung des mobilen Endgeräts und der bei Empfang des Nahfeld-Signals vorhandenen Geoposition des Ortungsgeräts 102 als Geopositionsangabe veranlassen.

Bei einer abermals anderen Variante kann die geographische Position des Ortungsgeräts 102 bestimmt werden anhand der geographischen Position eines mobilen Endgeräts 110, 116, dessen Nahfeld-Signale 126 das Ortungsgerät 102 empfängt. Vorzugsweise können die Nahfeld-Signale 126 die augenblicklichen GPS-Koordinaten des mobilen Endgeräts 110, 116 enthalten. Das Speichermodul kann dann eine Speicherung der Nutzerkennung des mobilen Endgeräts 110, 116 zusammen mit der geographischen Position als Geopositionsangabe veranlassen.

Wenn (nahezu kontinuierlich) eine Vielzahl von Nahfeld-Signalen 126 mit der gleichen Nutzerkennung empfangen wird, kann, wenn sich das Ortungsgerät 102 während des Empfangs dieser Nahfeld-Signale 126 bewegt (beispielsweise in einem sich bewegenden Fahrzeug befindet), als Geopositionsangabe ein Bewegungsprofil (Geoposition über der Zeit) des Ortungsgeräts 102 bestimmt werden (und insbesondere gespeichert werden). Wenn das Speichermodul feststellt, dass sich das Ortungsgerät 102 (nahezu) nicht bewegt hat, kann als Geopositionsangabe nur eine Geoposition gespeichert werden.

Wie bereits beschrieben wurde, kann mindestens ein Speicherkriterium vorgesehen sein. Das Speicherkriterium kann festlegen, ob eine Speicherung erfolgt und insbesondere ob eine Speicherung nicht erfolgt, beispielsweise abhängig von der Distanzangabe und/oder der Zeitdauerangabe, wie zuvor beschrieben wurde.

Alternativ oder zusätzlich können - anstelle der Speicherung einer tatsächlichen Distanz und/oder einer tatsächlichen Empfangsdauer und/oder einem tatsächlichen Empfangszeitpunkt - die jeweiligen tatsächlichen Werte zu jeweiligen vordefinierten Bereichen zugeordnet werden, wie bereits beschrieben wurde. In der Datenbank 106 kann dann nur der jeweils zugeordnete Bereich gespeichert werden.

Auch kann eine von der Zeitdauerangabe und der Distanzangabe abhängige Infektionswahrscheinlichkeitsfunktion vorgesehen sein, um einen (zuvor beschriebenen) Infektionswahrscheinlichkeitsfaktor zu bestimmen, abhängig von der tatsächlichen Distanz und Empfangsdauer. Dieser kann alternativ zu den vorherigen Angaben oder zusätzlich hierzu zu der jeweiligen Nutzerkennung gespeichert werden.

Insbesondere kann auch jedes mobile Endgerät 110, 116, umfassend eine anmeldungsgemäße Anwendung 118.1, 118.2, die vorgenannten Schritte ausführen.

In Schritt 203 erfolgt ein Aussenden, durch das Ortungsgerät 102, eines Nahfeld-Advertising-Signals 126, enthaltend zumindest ein Auswerteinformationsdatum, das zumindest eine potentielle Notwendigkeit einer Auswertung der mindestens einen gespeicherten Nutzerkennung angibt. Die Auswertung umfasst zumindest ein Vergleichen der mindestens einen in der Datenbank 106 des Ortungsgeräts 102 gespeicherten Nutzerkennung mit mindestens einer in der vorliegend zentralen Infektionsdatenbank 132 gespeicherten Infektionskennung, die einem eine Infektionskrankheit aufweisenden Nutzer zugeordnet ist. Bei anderen Varianten können dezentrale Infektionsdatenbanken vorgesehen sein, wobei die gespeicherten Infektionskennungen durch das Backendsystem aktualisiert werden können (wenn insbesondere neue Infektionskennungen vorliegen). Nachfolgend wird beispielhaft von einer zentralen Infektionsdatenbank ausgegangen, wobei sich die nachfolgenden Ausführungen auf dezentrale Infektionsdatenbanken übertragen lassen.

Das System 100 kann mindestens eine (nicht gezeigte) Vergleichseinheit und mindestens eine (nicht gezeigte) Detektionseinheit, die in der Vergleichseinheit intergiert sein kann, zur Durchführung des Vergleichsschritts umfassen.

Wie bereits beschrieben wurde, kann das mindestens eine Auswerteinformationsdatum ein Anfragedatum sein und/oder das mindestens eine Auswerteinformationsdatum kann nur angeben bzw. anzeigen, dass das Ortungsgerät 102 keine Auswertung veranlassen kann.

In Schritt 204 erfolgt ein Empfangen, durch das Ortungsgerät 102, eines Antwortsignals (insbesondere ein Nahfeld-Advertising-Signal 126) auf das ausgesendete Nahfeld-Advertising-Signal 126 von einem mobilen Auswerte-Endgerät 110, wobei das Antwortsignal ein Antwortdatum enthält (z.B. eine Antwort-Bestätigung des mobilen Auswerte-Endgeräts 110 auf das Anfragedatum zum Veranlassen der Auswertung oder ein Auswerteanfragedatum des mobilen Auswerte-Endgeräts 110, wobei das mobile Auswerte-Endgerät 110 das Ortungsgerät 102 mit dem Auswerteanfragedatum durch anfragt, ob eine Auswertung notwendig ist), das zumindest eine Bereitschaft des mobilen Auswerte-Endgeräts 110 zum Veranlassen der Auswertung der mindestens einen gespeicherten Nutzerkennung angibt.

In Schritt 205 erfolgt ein Übertragen der mindestens einen gespeicherten Nutzerkennung an das mobile Auswerte-Endgerät 110 zur Auswertung der mindestens einen gespeicherten Nutzerkennung, wenn das Ortungsgerät 102 das Antwortsignal erhält und die Auswertung notwendig ist.

Eine Auswertung kann beispielsweise dann tatsächlich notwendig sein, wenn mindestens eine Nutzerkennung in der Datenbank 106 gespeichert ist. Alternativ oder zusätzlich kann eine Auswertung bei einem Überschreiten einer bestimmten Nutzerkennungsanzahl erforderlich sein, also wenn beispielsweise (in einem Zeitraum X) mehr als Y (entspricht der bestimmten Nutzerkennungsanzahl) Nutzerkennungen in der Datenbank 106 gespeichert wurden. Auch kann eine Auswertung notwendig sein, wenn ein Zeitkriterium erfüllt ist, das beispielsweise angibt, dass eine Auswertung zumindest nach Ablauf einer bestimmten Zeitdauer (z.B. x Tage, x Stunden etc.) notwendig ist.

Die vorgenannten Schritte 201 bis 205 können zumindest teilweise parallel durchgeführt werden. Anschließend kann eine Auswertung erfolgen, wie nachfolgend näher beschrieben wird. Hierfür können das Auswerte-Endgerät 110 und das Backendsystem 130 über das Fernkommunikationsnetz 128 miteinander kommunizieren.

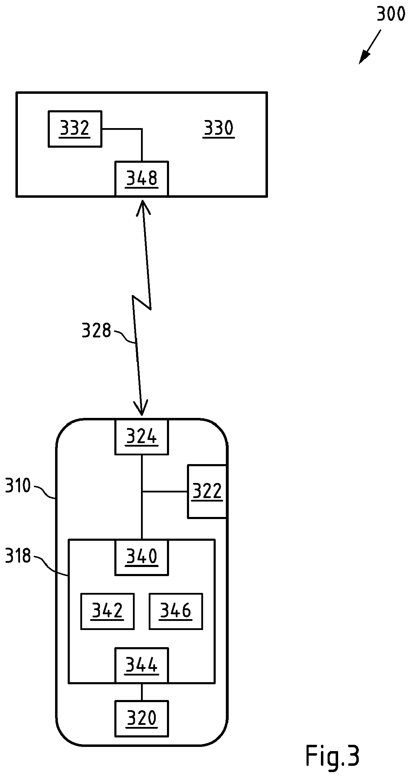

Die Figur 3 zeigt eine schematische Ansicht eines weiteren Ausführungsbeispiels eines Systems 300 gemäß der vorliegenden Anmeldung mit einem Ausführungsbeispiel einer Auswerte-Anwendung 318 gemäß der vorliegenden Anmeldung. Zur Vermeidung von Wiederholungen werden nachfolgend im Wesentlichen nur die Unterschiede zum vorherigen Ausführungsbeispiel beschrieben, und ansonsten wird auf die vorherigen Ausführungen verwiesen.

Insbesondere ist bei dem Ausführungsbeispiel nach Figur 3 vorgesehen, dass der vorbeschriebene Vergleichsschritt durch das mobile Auswerte-Endgerät 310 durchgeführt wird, wie nachfolgend erläutert wird. Anders ausgedrückt, erfolgt ein Durchführen des Vergleichsschritts lokal und insbesondere dezentral auf den jeweiligen mobilen Auswerte-Endgeräten 310 durch jeweils eine Vergleichseinheit in Form eines Vergleichsmoduls 344.

Auf dem mobilen Auswerte-Endgerät 310 ist eine anmeldungsgemäße Anwendung 318 installiert.

Das mobile Auswerte-Endgerät 310 weist mindestens eine Nahfeldschnittstelle 322 auf, beispielhaft eine zuvor beschriebene Bluetooth-Nahfeldschnittstelle 322. Darüber hinaus weist das mobile Auswerte-Endgerät 310 mindestens eine Fernkommunikationsschnittstelle 324 auf. Die Fernkommunikationsschnittstelle 324 ist zumindest eingerichtet zum Kommunizieren mit einer Fern-Kommunikationseinrichtung 348 des Backendsystems 330 über ein Fernkommunikationsnetz 328. Vorzugsweise kann eine Mobilfunktechnologie verwendet werden.

Die Anwendung 318 weist ein Empfangsmodul 340, ein optionales Speichermodul 342 (z.B. entsprechend dem zuvor beschriebenen Speichermodul der Anwendung des Ortungsgeräts), das Vergleichsmodul 344 (das ein nicht gezeigtes Detektionsmodul enthalten kann) und ein Ausgabemodul 346 auf. Die Funktionsweise wird nachfolgend beispielhaft beschrieben.

Das Backendsystem 330 umfasst die zentrale Infektionsdatenbank 332. In der Infektionsdatenbank 332 sind zumindest die Infektionskennungen gespeichert, also die Nutzerkennungen von infizierten Personen (vgl. z.B. Tabelle 2). Für jede Infektionskennung können als weitere Angaben die Infektionsart und/oder der Infektionszeitpunkt gespeichert werden.

Das mobile Auswerte-Endgerät 310 kann einen Infektionsdatensatz von dem Backendsystem 330 empfangen, über das Fern-Kommunikationsnetz 328. Beispielsweise kann ein Infektionsdatensatz in einer Broadcast-Nachricht enthalten sein, welches von sämtlichen mobilen Auswerte-Endgeräten 310 des Systems 300 empfangen und insbesondere, wie nachfolgend dargestellt, ausgewertet werden kann.

Der mindestens eine Infektionsdatensatz kann zumindest die Infektionskennungen enthalten, vorzugsweise zudem weitere Infektionsangaben, wie die Infektionsart und/oder den Infektionszeitpunkt.

Ferner kann das Auswerte-Endgerät 310 mindestens eine Nutzerkennung von einem (vorliegend nicht dargestellten Ortungsgerät, wie Ortungsgerät 102) empfangen, wie zuvor beschrieben wurde. Diese können beispielsweise in der Datenbank 320 für eine Auswertung zwischengespeichert werden.

Das Vergleichsmodul 344 ist insbesondere eingerichtet zum Durchführen des Vergleichsschritts. Insbesondere vergleicht das Vergleichsmodul 344 die erhaltenen Infektionskennungen mit den erhaltenen Nutzerkennungen. Vorzugsweise können in dem Vergleichsschritt zumindest die Infektionszeitangabe, die Empfangszeitpunktangabe und die Infektionskennung (insbesondere die Inkubationszeit der entsprechenden Infektionskrankheit) berücksichtigt werden.

Das mindestens eine Ausgabemodul 346 kann eingerichtet sein zum Bewirken eines Ausgebens einer Infektionsinformation, zumindest wenn in dem Vergleichsschritt durch ein (nicht dargestelltes Detektionsmodul) detektiert wird, dass die mindestens eine übertragene bzw. erhaltene Nutzerkennung mit mindestens einer in der zentralen Infektionsdatenbank 332 gespeicherten Infektionskennung korrespondiert, also insbesondere identisch ist, wie bereits beschrieben wurde. Es versteht sich, dass auch eine Infektionsinformation (z.B. "kein Kontakt mit einer infizierten Person") ausgegeben werden kann, wenn keine Korrespondenz festgestellt wurde.

Vorzugsweise kann das Ausgeben einer Infektionsinformation ein Übertragen, durch das mobile Auswerte-Endgerät 310, zumindest eines Vergleichsergebnisdatensatzes als Infektionsinformation an das (nicht gezeigte) Ortungsgerät umfassen, wobei insbesondere bei einer augenblicklich fehlenden Kommunikationsverbindung zu dem Ortungsgerät das mobile Auswerte-Endgerät 310 den Vergleichsergebnisdatensatz lokal in der Datenbank 320 zwischenspeichert und/oder an das Backendsystem 330 überträgt (wie zuvor beschrieben wurde).

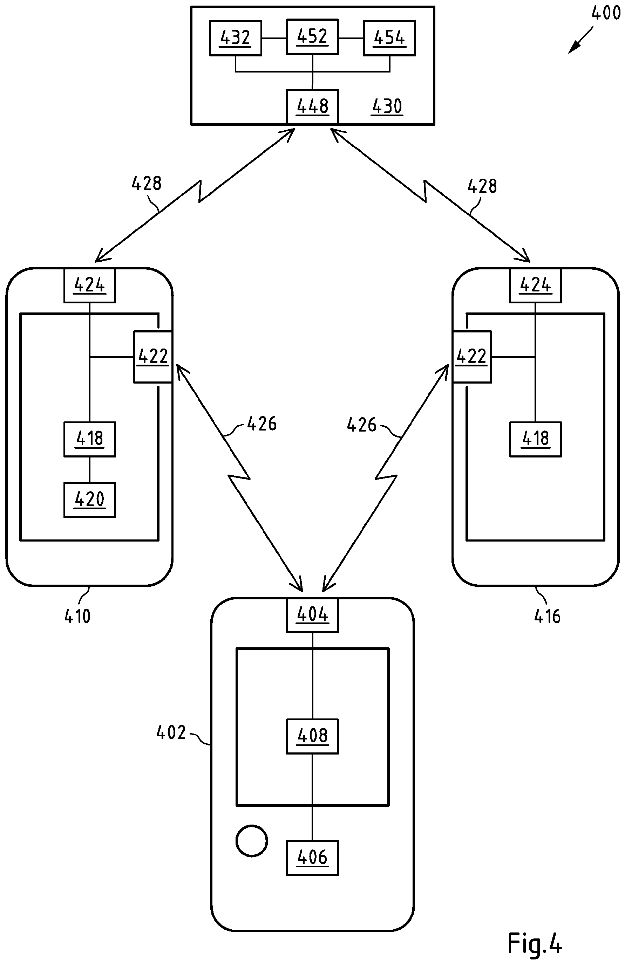

Die Figur 4 zeigt eine schematische Ansicht eines weiteren Ausführungsbeispiels eines Systems 400 gemäß der vorliegenden Anmeldung. Zur Vermeidung von Wiederholungen werden nachfolgend im Wesentlichen nur die Unterschiede zu den vorherigen Ausführungsbeispielen beschrieben und ansonsten auf die vorherigen Ausführungen verwiesen.

Im Vergleich zu dem Ausführungsbeispiel nach Figur 3 wird im vorliegenden Ausführungsbeispiel der Vergleichsschritt zentral durch das Backendsystem 430 durchgeführt. Hierfür weist das Backendsystem 430 vorliegend als Vergleichseinheit eine Vergleichseinrichtung 452 auf und insbesondere als Ausgabeeinheit die Kommunikationseinrichtung 448 auf.

Das mindestens eine Ortungsgerät 402, insbesondere die (einzelne) NahfeldSchnittstelle 404, ist eingerichtet zum Übertragen der mindestens einen gespeicherten Nutzerkennung (vorzugsweise sämtliche in der Datenbank 406 gespeicherte Angaben) an das mobile Auswerte-Endgerät 410 zur Auswertung der mindestens einen gespeicherten Nutzerkennung.

Vorzugsweise kann für das Übertragen, durch das Ortungsgerät 402, der mindestens einen gespeicherten Nutzerkennung ein dauerhafter Nahfeld-Kommunikationskanal zwischen dem Ortungsgerät 402 und dem mobilen Auswerte-Endgerät 410 hergestellt werden. Vorliegend wird zusätzlich zu der mindestens einen gespeicherten Nutzerkennung die Ortungsgeräte-Kennung des Ortungsgeräts 402 übertragen.

Ein Empfangsmodul der Anwendung 418 weist insbesondere auch eine Sendefunktion auf, um ein Aussenden eines Nutzerkennungsdatensatzes (enthaltend die mindestens eine übertragene Nutzerkennung, vorzugsweise sämtliche übertragene Angaben) durch die Fernkommunikationsschnittstelle 424 zu bewirken. Der Nutzerkennungsdatensatz kann vorliegend zusätzlich die übertragene Ortungsgeräte-Kennung des Ortungsgeräts 402 enthalten.

Nach einem Empfang eines Nutzerkennungsdatensatzes kann der (zuvor beschriebene) Vergleichsschritt durchgeführt werden, insbesondere durch die Vergleichseinrichtung 452.

Vorliegend wird beispielhaft - insbesondere zur Erläuterung der asynchronen Kommunikation - davon ausgegangen, dass das mobile Auswerte-Endgerät 410 nach Erstellung des Vergleichsergebnisses nicht mehr in Reichweite der Nachbereichsschnittstelle 404 des Ortungsgeräts 402 ist, also keine Übertragung eines Vergleichsergebnisdatensatzes über das Auswerte-Endgerät 410 an das Ortungsgerät 402erfolgen kann, enthaltend zumindest das Vergleichsergebnis (z.B. eine festgestellte Korrespondenz) und weitere Angaben, wie mindestens eine Handlungsempfehlung und/oder mindestens ein (vordefinierter) Notfallkontakt, .

In diesem Fall ordnet das Backendsystem 430 dem Vergleichsergebnisdatensatz insbesondere die zugehörige Ortungsgeräte-Kennung zu und speichert diese Daten temporär in einer (Ergebnis-)Datenbank 454 des Backendsystems 430.

Gelangt nun ein mobiles Endgerät 416 (zu einem späteren Zeitpunkt) in Reichweite der Nachbereichsschnittstelle 404 des Ortungsgeräts 402 und empfängt insbesondere ein Nahfeld-Advertising-Signal 426 des Ortungsgeräts 402, enthaltend zumindest die Ortungsgeräte-Kennung des Ortungsgeräts 402, so teilt das mobile Endgerät 416 dem Backendsystem 430 dies mit. Insbesondere erfolgt eine Mitteilung eines entsprechenden Kontakts durch Übertragen einer entsprechenden Nachricht über das Fernkommunikationsnetz 428.

Vorzugsweise kann vorgesehen sein, dass eine entsprechende Mitteilung durch das mobile Endgerät 416 nur dann erfolgt, wenn die erhaltene Ortungsgeräte-Kennung zu einer Ortungsgeräte-Kennung einer zuvor von dem Backendsystem 430 erhaltenen Liste korrespondiert, wie zuvor ausgeführt wurde.

Bei Erhalt einer entsprechenden Mitteilung kann das Backendsystem 430 prüfen, basierend auf der Ortungsgeräte-Kennung, ob Daten für eine Übertragung an das entsprechende Ortungsgerät 402 in der Ergebnis-Datenbank 454 des Backendsystems 430 gespeichert sind. Insbesondere kann nach einer Korrespondenz zwischen der erhaltenen Ortungsgeräte-Kennung und den jeweils einem gespeicherten Vergleichsergebnisdatensatz zugeordneten Ortungsgeräte-Kennungen gesucht werden.

Wenn eine Korrespondenz durch das Backendsystem 430 festgestellt wird, kann ein Übertragen des entsprechenden Vergleichsergebnisdatensatzes an das mobile Endgerät 416 erfolgen, um insbesondere eine Übertragung des Vergleichsergebnisdatensatzes durch das mobile Endgerät 416 an das entsprechende Ortungsgerät 402 zu bewirken.

Sofern sich das mobile Endgerät 416 nach Erhalt des Vergleichsergebnisdatensatzes noch immer in Reichweite der Nahfeldschnittstelle des Ortungsgeräts 402 befindet, kann ein Übertragen des Vergleichsergebnisdatensatzes an das Ortungsgerät 402 durchgeführt werden. Vorzugsweise kann hierfür, in zuvor beschriebener Weise, ein dauerhafter Nahfeld-Kommunikationskanal hergestellt werden.

Bei Erhalt des (gesamten) Vergleichsergebnisdatensatzes kann das Ortungsgerät 402 dies dem mobilen Endgerät 416 quittieren. Daraufhin kann durch das mobile Endgerät 416 ein Aussenden, an das Backendsystem 430, einer Bestätigungsnachricht über die vollständige Übertragung des Vergleichsergebnisdatensatzes an das Ortungsgerät 402 erfolgen. Anschließend kann ein Löschen des Vergleichsergebnisdatensatzes auf dem mobilen Endgerät 416 und/oder dem Backendsystem 430 erfolgen.

Wenn der Vergleichsergebnisdatensatz nicht oder unvollständig übertragen wurde, erfolgt keine (finale) Quittierung eines Erhalts des Vergleichsergebnisdatensatzes durch das Ortungsgerät 402. Wenn nur ein Teil des Vergleichsergebnisdatensatzes übertragen wurde, kann beispielsweise eine Teilquittierung durch das Ortungsgerät 402 über die erhaltenen Daten erfolgen.

Daraufhin kann durch das mobile Endgerät 416 ein Aussenden an das Backendsystem 430 einer Erwiderungsnachricht über die zumindest unvollständige (oder komplett erfolglose) Übertragung des Vergleichsergebnisdatensatzes an das Ortungsgeräts 402 erfolgen, wobei die Erwiderungsnachricht ein Informationsdatum über die nicht übertragenen Daten des Vergleichsergebnisdatensatzes enthält. Dies kann in dem Backendsystem 430, insbesondere in der genannten Datenbank 454 (zu dem entsprechenden Vergleichsergebnisdatensatz) protokolliert werden, wie zuvor beschrieben wurde.

Bei Empfang einer weiteren Mitteilung von einem mobilen Endgerät (z.B. 410, 416) über den Empfang der Ortungsgeräte-Kennung durch das Backendsystem 430 kann das Backendsystem 430 nur die noch nicht an das Ortungsgerät 402 übertragenen Daten des Vergleichsergebnisdatensatzes an das mobile Endgerät (z.B. 410, 416) übertragen. Dies kann solange weiter durchgeführt werden, bis der gesamte Vergleichsergebnisdatensatz an das entsprechende Ortungsgerät 402 übertragen wurde (und/oder bis eine bestimmte Zeitdauer abgelaufen ist).

Auf dem mobilen Endgerät 416 kann eine entsprechende Anwendung 418 gespeichert sein, die die vorbeschriebenen Schritte auf dem Endgerät 416 steuert.

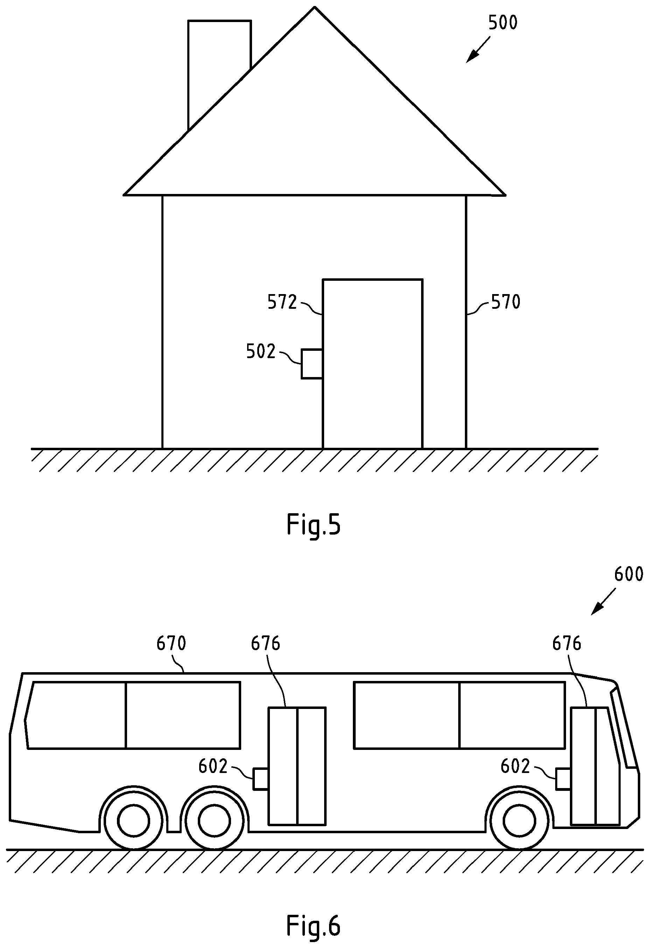

Die Figur 5 zeigt eine schematische Ansicht eines weiteren Ausführungsbeispiels eines Systems 500 gemäß der vorliegenden Anmeldung. Zur Vermeidung von Wiederholungen werden nachfolgend im Wesentlichen nur die Unterschiede zu den vorherigen Ausführungsbeispielen beschrieben, und ansonsten wird auf die vorherigen Ausführungen verwiesen.

Das mindestens eine Ortungsgerät 502 ist ein stationäres Ortungsgerät 502, das einer bestimmten Entität 570 fest bzw. dauerhaft zugeordnet ist.

Die Entität 570 ist vorliegend ein Gebäude 570. Beispielsweise an mindestens einer Zugangsordnung 572 und/oder mindestens einem Eingang/Ausgang 572 des Gebäudes 570 (z.B. Bahnhof, Krankenhaus, Bürogebäude, Pflegeheim) kann das Ortungsgerät 502 fest (also dauerhaft unbeweglich) installiert sein. Die Installationsposition kann bei der Registrierung des Ortungsgeräts 502 registriert werden und/oder in dem Ortungsgerät 502 hinterlegt sein.

Die Figur 6 zeigt eine schematische Ansicht eines weiteren Ausführungsbeispiels eines Systems 600 gemäß der vorliegenden Anmeldung. Zur Vermeidung von Wiederholungen werden nachfolgend im Wesentlichen nur die Unterschiede zu den vorherigen Ausführungsbeispielen beschrieben, und ansonsten wird auf die vorherigen Ausführungen verwiesen.

Das mindestens eine Ortungsgerät 602 ist ein stationäres Ortungsgerät 602, das einer bestimmten Entität 670 zugeordnet ist. Die Entität 670 ist vorliegend ein Fahrzeug 670. Beispielsweise an mindestens einer Zugangsordnung 676 (insbesondere an sämtlichen Zugangsordnungen 676) und/oder mindestens einem Eingang/Ausgang 676 (insbesondere an sämtlichen Eingängen/Ausgängen 676) des Fahrzeugs 670 (z.B. Bus, Schienenfahrzeug etc.) kann das Ortungsgerät 602 fest installiert sein, also dauerhaft und unbeweglich in Relation zu der Entität 670 montiert sein. Die Installationsposition kann bei der Registrierung registriert werden und/oder in dem Ortungsgerät 602 hinterlegt sein.

Es versteht sich, dass ein Ortungsgerät auch an anderen Positionen eines Gebäudes und/oder eines Fahrzeugs stationär installiert sein kann.

Insbesondere kann anmeldungsgemäß eine Möglichkeit zur Selbstüberprüfung via ein Ortungsgerät über einen Kontakt mit infizierten Personen (anonym und ohne Tracking von Bewegungsdaten von Personen) bereitgestellt werden. Der vorbeschriebene anmeldungsgemäße Lösungsansatz umfasst insbesondere:
(1) Eine App eines Ortungsgeräts speichert empfangene, anonymisierte Personen-IDs bzw. Nutzerkennungen anderer Personen bzw. der zugeordneten Endgeräte in einem definierten Umkreis/Dauer.
(2) In einer zentralen Infektionsdatenbank wird im Falle einer Infizierung die anonymisierte Personen-ID bzw. Nutzerkennung einer infizierten Person hinterlegt, also die Infektionskennung.
(3) Über die App kann dann individuell die Auswertung/Abfrage erfolgen, ob auf dem Ortungsgerät eine entsprechende Personen-ID in der Vergangenheit empfangen wurde und eine Info bzgl. Nähe, Dauer (Exposure Time) etc. erfolgen.

## Patentansprüche

1. Verfahren zum Rekonstruieren von Infektionsketten, mit den Schritten:
- Empfangen, durch ein Ortungsgerät (102, 402, 502, 602), mindestens eines Nahfeld-Signals (126, 426) mindestens eines in Empfangsreichweite des Ortungsgeräts (102, 402, 502, 602) befindlichen mobilen Endgeräts (110, 116, 310, 410, 416) oder eines weiteren Ortungsgeräts (102, 402, 502, 602),
- Speichern mindestens einer Nutzerkennung in einer Datenbank (106, 306, 406) des Ortungsgeräts (102, 402, 502, 602), wobei die Nutzerkennung in dem mindestens einen empfangenen Nahfeld-Signal (126, 426) enthalten ist,
- Aussenden, durch das Ortungsgerät (102, 402, 502, 602), eines Nahfeld-Advertising-Signals (126, 426), enthaltend zumindest ein Auswerteinformationsdatum, das zumindest eine potentielle Notwendigkeit einer Auswertung der mindestens einen gespeicherten Nutzerkennung angibt, wobei die Auswertung zumindest ein Vergleichen der mindestens einen in der Datenbank (106, 306, 406) des Ortungsgeräts (102, 402, 502, 602) gespeicherten Nutzerkennung mit mindestens einer in einer Infektionsdatenbank (132, 332, 432) gespeicherten Infektionskennung umfasst, die einem eine Infektionskrankheit aufweisenden Nutzer zugeordnet ist,
- Empfangen, durch das Ortungsgerät (102, 402, 502, 602), eines Antwortsignals auf das ausgesendete Nahfeld-Advertising-Signal von einem mobilen Auswerte-Endgerät (110, 310, 410), wobei das Antwortsignal ein Antwortdatum enthält, das zumindest eine Bereitschaft des mobilen Auswerte-Endgeräts (110, 310, 410) zum Veranlassen der Auswertung der mindestens einen gespeicherten Nutzerkennung angibt, und
- Übertragen der mindestens einen gespeicherten Nutzerkennung an das mobile Auswerte-Endgerät (110, 310, 410) zur Auswertung der mindestens einen gespeicherten Nutzerkennung, wenn das Ortungsgerät (102, 402, 502, 602) das Antwortsignal erhält und die Auswertung notwendig ist.

2. Verfahren nach Anspruch 1, **dadurch gekennzeichnet, dass**
- das Auswerteinformationsdatum ein Anfragedatum ist, wobei mit dem Anfragedatum durch das Ortungsgerät (102, 402, 502, 602) ein mobiles Auswerte-Endgerät (110, 310, 410) für die Auswertung angefragt wird, und
- das Antwortdatum eine Antwort-Bestätigung eines mobilen Auswerte-Endgeräts (110, 310, 410) auf das Anfragedatum zum Veranlassen der Auswertung ist.

3. Verfahren nach Anspruch 1, **dadurch gekennzeichnet, dass**
- das Auswerteinformationsdatum nur angibt, dass das Ortungsgerät (102, 402, 502, 602) keine Auswertung veranlassen kann, und
- das Antwortdatum ein Auswerteanfragedatum eines mobilen Auswerte-Endgeräts (110, 310, 410) ist, wobei mit dem Auswerteanfragedatum durch das mobile Auswerte-Endgerät (110, 310, 410) das Ortungsgerät (102, 402, 502, 602) angefragt wird, ob eine Auswertung notwendig ist, und
- insbesondere vor einem Übertragen der mindestens einen gespeicherten Nutzerkennung an das mobile Auswerte-Endgerät (110, 310, 410) das Ortungsgerät (102, 402, 502, 602) eine Bestätigungsantwort auf das Antwortsignal sendet, dass die Auswertung gewünscht ist.

4. Verfahren nach einem der vorherigen Ansprüche, **dadurch gekennzeichnet, dass**
- für ein Übertragen, durch das Ortungsgerät (102, 402, 502, 602), der mindestens einen gespeicherten Nutzerkennung ein dauerhafter Nahfeld-Kommunikationskanal zwischen dem Ortungsgerät (102, 402, 502, 602) und dem mobilen Auswerte-Endgerät (110, 310, 410) hergestellt wird.

5. Verfahren nach einem der vorherigen Ansprüche, **dadurch gekennzeichnet, dass** das Auswerten der mindestens einen übertragenen Nutzerkennung ferner die Schritte umfasst:
- Vergleichen, in einem Vergleichsschritt, der mindestens einen übertragenen Nutzerkennung mit mindestens einer in der Infektionsdatenbank (132, 332, 432) gespeicherten Infektionskennung, die einem eine Infektionskrankheit aufweisenden Nutzer zugeordnet ist,
- Detektieren einer Korrespondenz zwischen der mindestens einen übertragenen Nutzerkennung und der mindestens einen gespeicherten Infektionskennung und
- Übertragen, basierend auf der detektierten Korrespondenz, einer Infektionsinformation an das Ortungsgerät (102, 402, 502, 602).

6. Verfahren nach Anspruch 5, **dadurch gekennzeichnet, dass** die Infektionsdatenbank (132, 332, 432) in einem Backendsystem (130, 330, 430) angeordnet ist, wobei das Verfahren ferner umfasst:
- Übertragen, durch das mobile Auswerte-Endgerät (110, 310, 410), eines Nutzerkennungsdatensatzes an das Backendsystem (130, 330, 430), wobei der Nutzerkennungsdatensatz zumindest die mindestens eine übertragene Nutzerkennung enthält,
- Durchführen, durch das Backendsystem (130, 330, 430), des Vergleichsschritts, und
- Übertragen, durch das Backendsystem (130, 330, 430), zumindest eines Vergleichsergebnisdatensatzes als Infektionsinformation an das Ortungsgerät (102, 402, 502, 602).

7. Verfahren nach Anspruch 6, **dadurch gekennzeichnet, dass**
- die mindestens eine übertragene Nutzerkennung zusammen mit einer Ortungsgeräte-Kennung des Ortungsgeräts (102, 402, 502, 602) übertragen wird,
- der auf der mindestens einen übertragenen Nutzerkennung basierende Nutzerkennungsdatensatz zumindest die Ortungsgeräte-Kennung enthält,
- dem auf dem übertragenen Nutzerkennungsdatensatz basierende Vergleichsergebnisdatensatz die Ortungsgeräte-Kennung zugeordnet wird und
- das Übertragen zumindest des Vergleichsergebnisdatensatzes an das Ortungsgerät (102, 402, 502, 602) über mindestens ein mobiles Endgerät (110, 116, 310, 410, 416) auf der Ortungsgeräte-Kennung basiert, die dem Vergleichsergebnisdatensatz zugeordnet ist.

8. Verfahren nach Anspruch 7, **dadurch gekennzeichnet, dass** das Verfahren ferner umfasst:
- Empfangen, durch das mobile Endgerät (110, 116, 310, 410, 416), eines Nahfeld-Advertising-Signals des Ortungsgeräts (102, 402, 502, 602), enthaltend zumindest die Ortungsgeräte-Kennung des Ortungsgeräts (102, 402, 502, 602),
- Mitteilen, durch das mobile Endgerät (110, 116, 310, 410, 416), eines Empfangs der Ortungsgeräte-Kennung dem Backendsystem (130, 330, 430),
- Übertragen, durch das Backendsystem (130, 330, 430), des Vergleichsergebnisdatensatzes, der der mitgeteilten Ortungsgeräte-Kennung zugeordnet ist, an das mobile Endgerät (110, 116, 310, 410, 416), und
- zumindest teilweises Übertragen, durch das mobile Endgerät (110, 116, 310, 410, 416), des Vergleichsergebnisdatensatzes an das Ortungsgerät (102, 402, 502, 602).

9. Verfahren nach Anspruch 8, **dadurch gekennzeichnet, dass** das Verfahren ferner umfasst:
- Aussenden, durch das mobile Endgerät (110, 116, 310, 410, 416), an das Backendsystem (130, 330, 430) einer Bestätigungsnachricht über die vollständige Übertragung des Vergleichsergebnisdatensatzes an das Ortungsgerät (102, 402, 502, 602),
und/oder
- Aussenden, durch das mobile Endgerät (110, 116, 310, 410, 416), an das Backendsystem (130, 330, 430) einer Erwiderungsnachricht über die zumindest unvollständige Übertragung des Vergleichsergebnisdatensatzes an das Ortungsgerät (102, 402, 502, 602), wobei die Erwiderungsnachricht ein Informationsdatum über die nicht übertragenen Daten des Vergleichsergebnisdatensatzes enthält.

10. Verfahren nach Anspruch 9, **dadurch gekennzeichnet, dass**
- das Backendsystem (130, 330, 430) bei einem Vergleichsergebnisdatensatz zumindest protokolliert, welche Daten noch nicht an das zugehörige Ortungsgerät übertragen wurden, und
- bei Empfang einer Mitteilung von einem mobilen Endgerät (110, 116, 310, 410, 416) über den Empfang der Ortungsgeräte-Kennung das Backendsystem (130, 330, 430) nur die noch nicht an das Ortungsgerät (102, 402, 502, 602) übertragenen Daten des Vergleichsergebnisdatensatzes an das mobile Endgerät (110, 116, 310, 410, 416) überträgt.

11. Verfahren nach einem der Ansprüche 6 bis 10, **dadurch gekennzeichnet, dass**
- nach einem Empfang, durch das mobile Auswerte-Endgerät (110, 310, 410), der mindestens einen übertragenen Nutzerkennung und bei einer augenblicklich fehlenden Kommunikationsverbindung zu dem Backendsystem (130, 330, 430) das mobile Auswerte-Endgerät (110, 310, 410) die mindestens eine übertragene Nutzerkennung lokal zwischenspeichert, und
- der auf der mindestens einen zwischengespeicherten Nutzerkennung basierende Nutzerkennungsdatensatz, durch das mobile Auswerte-Endgerät (110, 310, 410), bei einer Detektion einer Kommunikationsverbindung zu dem Backendsystem (130, 330, 430) übertragen wird.

12. Verfahren nach einem der vorherigen Ansprüche, **dadurch gekennzeichnet, dass** das Verfahren ferner umfasst:
- Übertragen, durch das Backendsystem (130, 330, 430), eines Infektionsdatensatzes zumindest an das mobile Auswerte-Endgerät (110, 310, 410), an das die mindestens eine Nutzerkennung übertragen wurde,
- Durchführen, durch das mobile Auswerte-Endgerät (110, 310, 410), des Vergleichsschritts, und
- Übertragen, durch das mobile Auswerte-Endgerät (110, 310, 410), zumindest eines Vergleichsergebnisdatensatzes als Infektionsinformation an das Ortungsgerät (102, 402, 502, 602),
- wobei insbesondere bei einer augenblicklich fehlenden Kommunikationsverbindung zu dem Ortungsgerät (102, 402, 502, 602) das mobile Auswerte-Endgerät (110, 310, 410) den Vergleichsergebnisdatensatz lokal zwischenspeichert und/oder an das Backendsystem (130, 330, 430) überträgt.

13. Verfahren nach einem der vorherigen Ansprüche, **dadurch gekennzeichnet, dass das** Verfahren ferner umfasst:
- Registrieren eines Ortungsgeräts (102, 402, 502, 602) in einem zentralen Register durch Speichern zumindest der Ortungsgeräte-Kennung des Ortungsgeräts (102, 402, 502, 602) in dem zentralen Register,
- wobei das Registrieren ein Ausgeben, durch das Ortungsgerät (102, 402, 502, 602), der Ortungsgeräte-Kennung umfasst, und
- wobei das Registrieren ein Übertragen der ausgegebenen Ortungsgeräte-Kennung durch ein weiteres Gerät (110, 116, 310, 410, 416) an das zentrale Register umfasst.

14. Verfahren nach einem der vorherigen Ansprüche, **dadurch gekennzeichnet, dass**
- das Ortungsgerät (102, 402, 502, 602) einem Nutzer zugeordnet ist, und
- bei einer Infektion des Nutzers ein verifiziertes Endgerät (110, 116, 310, 410, 416) die Ortungsgeräte-Kennung der Infektionsdatenbank (132, 332, 432) als verifizierte Infektionskennung meldet,
und/oder
- bei einer Infektion des Nutzers ein nicht-verifiziertes Endgerät (110, 116, 310, 410, 416) die Ortungsgeräte-Kennung der Infektionsdatenbank (132, 332, 432) als nicht-verifizierte Infektionskennung meldet, wobei insbesondere durch ein verifiziertes Endgerät (110, 116, 310, 410, 416) eine nicht-verifizierte Infektionskennung verifiziert werden kann.

15. Verfahren nach einem der vorherigen Ansprüche 1 bis 13, **dadurch gekennzeichnet, dass**
- das Ortungsgerät (102, 402, 502, 602) ein stationäres Ortungsgerät (102, 402, 502, 602) ist, das einer bestimmten Entität (570, 670) zugeordnet ist,
- wobei die Entität (570, 670) insbesondere ein Gebäude (570) oder ein Fahrzeug (670) ist.

16. Ortungsgerät (102, 402, 502, 602), umfassend:
- mindestens eine Nahfeldschnittstelle (104, 404), eingerichtet zum Empfangen, mindestens eines Nahfeld-Signals (126, 426) mindestens eines in Empfangsreichweite des Ortungsgeräts (102, 402, 502, 602) befindlichen mobilen Endgeräts (110, 116, 310, 410, 416) oder eines weiteren Ortungsgeräts (102, 402, 502, 602),
- mindestens eine Datenbank (106, 406), eingerichtet zum Speichern mindestens einer Nutzerkennung, wobei die Nutzerkennung in dem mindestens einen empfangenen Nahfeld-Signal (126, 426) enthalten ist,
- wobei die Nahfeldschnittstelle (104, 404) eingerichtet ist zum Aussenden eines Nahfeld-Advertising-Signals (126, 426), enthaltend zumindest ein Auswerteinformationsdatum, wobei das Antwortsignal ein Antwortdatum enthält, das zumindest eine Bereitschaft des mobilen Auswerte-Endgeräts (110, 310, 410) zum Veranlassen der Auswertung der mindestens einen gespeicherten Nutzerkennung angibt, wobei die Auswertung zumindest ein Vergleichen der mindestens einen in der Datenbank (106, 406) des Ortungsgeräts (102, 402, 502, 602) gespeicherten Nutzerkennung mit mindestens einer in einer Infektionsdatenbank (132, 332, 432) gespeicherten Infektionskennung umfasst, die einem eine Infektionskrankheit aufweisenden Nutzer zugeordnet ist,
- wobei die Nahfeldschnittstelle (104, 404) eingerichtet ist zum Empfangen eines Antwortsignals auf das ausgesendete Nahfeld-Advertising-Signal von einem mobilen Auswerte-Endgerät (110, 310, 410), wobei das Antwortsignal ein Antwortdatum enthält, das zumindest eine Bereitschaft des mobilen Auswerte-Endgeräts zum Veranlassen der Auswertung der mindestens einen gespeicherten Nutzerkennung angibt, und
- wobei die Nahfeldschnittstelle (104, 404) eingerichtet ist zum Übertragen der mindestens einen gespeicherten Nutzerkennung an das mobile Auswerte-Endgerät (110, 310, 410) zur Auswertung der mindestens einen gespeicherten Nutzerkennung, wenn das Ortungsgerät (102, 402, 502, 602) das Antwortsignal erhält und die Auswertung notwendig ist.

17. Anwendung (118, 318, 418) zum Rekonstruieren von Infektionsketten, insbesondere in Form einer durch einen Prozessor eines mobilen Auswerte-Endgeräts (110, 310, 410) ausführbaren Softwareanwendung (118, 318, 418), zur Installation auf dem mobilen Auswerte-Endgerät (110, 310, 410), umfassend:
- mindestens ein Empfangsmodul (340), eingerichtet zum Erhalten mindestens einer Nutzerkennung von einem Ortungsgerät (102, 402, 502, 602) nach Anspruch 16,
- mindestens ein Detektionsmodul, eingerichtet zum Detektieren, in einem durch ein Vergleichsmodul 344) durchgeführten Vergleichsschritt, dass die mindestens eine erhaltene Nutzerkennung des Ortungsgeräts (102, 402, 502, 602) korrespondiert mit mindestens einer in einer Infektionsdatenbank (132, 332, 432) gespeicherten Infektionskennung, die einem eine Infektionskrankheit aufweisenden Nutzer zugeordnet ist, und
- mindestens ein Ausgabemodul (346), eingerichtet zum Bewirken eines Ausgebens einer Infektionsinformation, zumindest wenn in dem Vergleichsschritt mindestens ein Korrespondieren detektiert wird.
